# EUROPEAN PATENT APPLICATION

(11) **EP 4 365 598 A1**
(43) Date of publication of application: **08.05.2024**
(21) Application number: 23155673.9
(22) Date of filing: 08.02.2023
(51) Int. Cl.: G01N 33/74

(54) **RAPID OPTIMIZATION OF ORAL HEART FAILURE THERAPIES HELPED BY NT-PROBNP TESTING**

(30) Priority: 07.11.2022 US 202263423191 P; 11.01.2023 US 202363438341 P
(71) Applicant: Hôpitaux Universitaires Saint-Louis-Lariboisière, 75010 Paris (FR); Université de Paris Cité, 75006 Paris (FR); Inserm, 75654 Paris Cedex 13 (FR); Momentum Research Inc., Durham, NC 27707 (US)
(72) Inventor: Cotter, Gad, Durham, NC 27707 (US); Davison, Beth, Durham, NC 27707 (US); Mebazaa, Alexandre, 75006 Paris (FR)
(74) Representative: Altmann Stößel Dick Patentanwälte PartG mbB

(57) **Abstract**

The present invention relates to a method for assessing heart failure therapy in a patient who has been hospitalized for acute heart failure, said method comprising the steps of a) determining the amount of a BNP-type peptide, such as NT-proBNP, in a baseline sample that has been obtained prior to discharge of the patient from hospital, b) determining the amount of the BNP-type peptide in at least one further sample wherein said at least one further sample has been obtained after discharge of the patient, and comparing the amount in the baseline sample determined in step a) to the amount in the at least one further sample determined in step b). The present invention further relates to a device for assessing heart failure therapy as well as to a method of treating a patient who has been hospitalized for acute heart failure.

## Description

### Overview

The present invention relates to a method for assessing heart failure therapy in a patient who has been hospitalized for acute heart failure, said method comprising the steps of a) determining the amount of a BNP-type peptide, such as NT-proBNP, in a baseline sample that has been obtained prior to discharge of the patient from hospital, b) determining the amount of the BNP-type peptide in at least one further sample, wherein said at least one further sample has been obtained after discharge of the patient, and comparing the amount in the baseline sample determined in step a) to the amount in the at least one further sample determined in step b). The present invention further relates to a device for assessing heart failure therapy as well as to a method of treating a patient who has been hospitalized for acute heart failure.

### Background

Acute heart failure (AHF) is a major contributor to the morbidity and mortality of patients with heart failure (HF) (1). Despite this increased risk, the vast majority of AHF patients are not closely followed and are substantially undertreated during the first few months after discharge from an AHF admission - a "vulnerable period" during which most of the adverse events occur (2-7). Some authors have retrospectively examined the association of more follow-up and quicker up-titration of medications after an AHF event, but with mixed results (8-1416). Based on this lack of evidence from prospective randomized studies, recent European guidelines for the treatment of HF recommend some follow-up of patients after an AHF admission and initiation of recommended therapies, but the level of evidence for this recommendation is low (C) (17).

The Safety, Tolerability and efficacy of Rapid Optimization, helped by NT-proBNP testinG, of Heart Failure therapies (STRONG-HF) study was a randomized, prospective clinical trial, designed to assess the safety and efficacy of rapid up-titration of medical therapy including beta-blockers (BB)s; angiotensin converting enzyme inhibitors (ACEi), angiotensin receptor blockers (ARB), or angiotensin receptor neprilysin inhibitors (ARNi), and mineralocorticoid receptor antagonists (MRA) prior to discharge from an AHF admission and during the following weeks (ClinicalTrials.gov NCT03412201). The safety of up-titration was guided by physical examination and laboratory evaluations including NTproBNP. Guideline-recommended oral HF medications for patients in the "high intensity care" arm were up-titrated to half optimal doses at discharge and to full optimal doses at 2 weeks postdischarge with safety visits 1 week after any up-titration and follow-up visits at 6 weeks and 3 months. At each visit, patients were assessed by physical examination for congestion and blood tests including NT-proBNP measurements (18,19).

In some of the patients that participated in the STRONG-HF study, a rise in NT-proBNP was observed after discharge. The usual response in the scientific community is that in response to a rise in NT-proBNP, there is a perception that there is a need to increase the dose of one or more components of GDMT (guideline directed medical therapy) which is expected to lead to the reduction of NT-proBNP level.

In the context of the studies underlying the present invention, a different hypothesis has been proven that in case of a rise in NT-proBNP, by increasing diuretics and maintaining or decreasing GDMT components, NT-proBNP levels will drop mirroring the improvement in the patient status and outcome.

In patients with acute heart failure after hospital discharge in which NT-proBNP increases in the discharge period, this indicates worsening of patients' heart failure status with very high sensitivity (vs clinical data). In these patients, the NT-proBNP rise after discharge indicates a change of therapy by increasing the dose of diuretics associated with maintaining or slight reduction of the doses of one or more components of the guideline directed medical therapy (GDMT), see e.g. patients 1 to 15 in the Examples section. Without this change of therapy, the condition of the patient might worsen (see e.g. patients 16 to 18 the Examples section).

Accordingly, the present invention, *inter alia,* relates to methods for assessing heart failure therapy in patients who have hospitalized for, i.e. due to acute heart failure as well as to methods of treating such patients. The methods of the present invention, if applied, allow for reduction in NT-proBNP levels and, thus, to an improvement of patient status and outcomes. For example, the risk of all cause death or heart failure readmission can be reduced.

### Brief Summary of the present invention

In a first aspect, the present invention relates to a method for assessing heart failure therapy in a patient who has been hospitalized for acute heart failure, said method comprising the steps of
a) determining the amount of a BNP-type peptide, such as NT-proBNP, in a baseline sample, wherein said baseline sample has been obtained prior to discharge of the patient from hospital,
b) determining the amount of the BNP-type peptide in at least one further sample wherein said at least one further sample has been obtained after discharge of the patient, and
c) comparing the amount in the baseline sample determined in step a) to the amount in the at least one further sample determined in step b).

Preferably an in increase of the amount of the BNP-type peptide in the at least one further sample as compared to the amount in the baseline sample is indicative for a patient who is eligible to an adaptation of the heart failure therapy. More preferably, an increase, an unchanged amount, or a decrease of less than 10% of the amount of the BNP-type peptide in the at least one further sample as compared to the amount in the baseline sample is indicative for a patient who is eligible to an adaptation of the heart failure therapy.

In a second aspect, the present invention further relates to a computer-implemented method for assessing heart failure therapy in a patient who has been hospitalized for acute heart failure, said method comprising the steps of
a) receiving, at a processing unit, a value for the amount of a BNP-type peptide in a baseline sample, wherein said baseline sample has been obtained prior to discharge of the patient from hospital,
b) receiving, at the processing unit, a value determining the amount of the BNP-type peptide in at least one further sample wherein said at least one further sample has been obtained after discharge of the patient, and
c) carrying out at the processing unit a comparison of the amount in the baseline sample determined in step a) to the amount in the at least one further sample determined in step b).

Preferably, an increase of the amount of the BNP-type peptide in the at least one further sample as compared to the amount in the baseline sample is indicative for a patient who is eligible to an adaptation of the heart failure therapy. More preferably, an increase, and unchanged amount, or a decrease of less than 10% of the amount of the BNP-type peptide in the at least one further sample as compared to the amount in the baseline sample is indicative for a patient who is eligible to an adaptation of the heart failure therapy.

In a third aspect, the present invention further relates to a method of treating a patient who has been hospitalized for acute heart failure, said method comprising the steps of
a) determining the amount of a BNP-type peptide, such as NT-proBNP, in a baseline sample, wherein said baseline sample has been obtained prior to discharge of the patient from hospital,
b) determining the amount of the BNP-type peptide in at least one further sample wherein said at least one further sample has been obtained after discharge of the patient,
c) comparing the amount in the baseline sample determined in step a) to the amount in the at least one further sample determined in step b),
d) identifying a patient as being eligible to an adaptation of the heart failure therapy, preferably based the results of step c),
e) treating the identified patient with the adapted heart failure therapy.

The heart failure therapies according to the present invention are oral heart failure therapies. Preferably, oral heart failure therapy comprises administration of at least one diuretic and at least one component of the guideline directed medical therapy (GDMT).

Preferably, the adaptation of the heart failure therapy (for the first, second and third aspect) is an increase of the dose of at least one diuretic, while not increasing of the dose of one or more components of guideline directed medical therapy (GDMT). More preferably, the adaptation of the therapy is an increase of the dose of at least one diuretic, while not increasing of the dose of one or more components of guideline directed medical therapy (GDMT) immediately. Thus, an increase of the dose is delayed. In addition, the dose of one or more components may be also reduced.

Thus, steps d) and e) of the method of treatment are preferably as follows:
d) identifying a patient as being eligible to an increase of the dose of at least one diuretic, but not to an (preferably immediate) increase of the dosage of guideline directed medical therapy (GDMT), preferably based the results of step c),
e) treating the identified patient with an increased dose of at least one diuretic, while maintaining the dose of one or more of the guideline directed medical therapies (GDMT).

In the methods of the present invention therapy is adapted as set forth above, if the amount of the marker is i) increased, ii) unchanged, or iii) decreased by less than 10% as compared to the amount in the baseline sample.

If the amount of the biomarker is decreased by more than 10%, preferably the dose of the diuretic is maintained while the dose of the one or more components of the guideline directed medical therapies (GDMT) can be increased.

The present invention further relates to the *in vitro* use of
i. at least one BNP-type peptide or
ii. at least one detection agent for the at least one BNP-type peptide
in a baseline sample and in at least one further sample obtained from a patient who has been hospitalized for acute heart failure for assessing heart failure therapy in said patient,
wherein said baseline sample has been obtained prior to discharge of the patient from hospital, and said at least one further sample has been obtained after discharge of the patient.

The present invention relates to a device for assessing heart failure therapy, said device comprising a processing unit, and a computer program including computer-executable instructions, wherein said instructions, when executed by the processing unit, cause the processing unit to perform the computer-implemented method of the present invention.

In an embodiment of the methods or the use of the present invention, the sample is a blood, serum, or plasma sample or interstitial fluid.

In another embodiment of the methods or the use of the present invention, the sample is interstitial fluid.

In a preferred embodiment of the methods or the use of the present invention, the baseline sample has been obtained from the patient within two days prior to the discharge of the patient from the hospital.

In a preferred embodiment of the methods or the use of the present invention, the at least one further sample has been obtained from the patient about 1, about 2, about 3, and/or about 6 weeks after discharge from hospital.

In an embodiment of the methods or the use of the present invention, the BNP-type peptide is NT-proBNP or BNP, in particular NT-proBNP.

In an embodiment of the methods or the use of the present invention, a decrease of less than 10%, an unchanged amount, or an increase of the amount of the BNP-type peptide in the at least one further sample of at least 10%, such as at least 20%, at least 30%, at least 40% or at least 50% as compared to the amount in the baseline sample is indicative for a patient who is eligible to an adapted heart failure therapy, in particular to an increase of the dose of at least one diuretic, but not to an increase of the dose of one or more guideline directed medical therapies (GDMT).

In an embodiment of the methods or the use of the present invention, the patient has preserved ejection fraction or wherein the patient has a reduced ejection fraction.

In another embodiment of the methods or the use of the present invention, the patient has a preserved ejection fraction.

In an embodiment of the methods or the use of the present invention, the guideline directed medical therapy is the administration of one or more of the following medicaments
i. at least one beta-blocker (BB);
ii. at least one angiotensin converting enzyme inhibitor (ACEi), at least one angiotensin receptor blocker (ARB), and/or at least one angiotensin receptor neprilysin inhibitor (ARNi),
iii. at least one mineralocorticoid receptor antagonists (MRA) and/or
iv. at least one SGLT2 inhibitor.

The medicaments under i, ii, iii and iv are herein also referred to as components of GDMT. For example, the administration of a beta blocker is a component of GDMT.

The preferred components are components i), ii) and/or iii).

The adapted therapy as referred to herein reduces the risk of negative outcomes. For example, the adapted therapy reduces the risk of worsening of heart failure (as compared ot a therapy that is not adapted).

### Definition and detailed overview on the present invention

As set forth above, the present invention relates to a method for assessing heart failure therapy in a patient who has been hospitalized for acute heart failure (first aspect).

The method of the present invention, preferably, is an ex vivo or in vitro method. Moreover, it may comprise steps in addition to those explicitly mentioned above. For example, further steps may relate to sample pre-treatments or evaluation of the results obtained by the method. The method may be carried out manually or assisted by automation. Preferably, step (a) and/or (b) may in total or in part be assisted by automation, e.g., by a suitable robotic and sensory equipment for the measurement in steps (a) or a computer-implemented identification in step (c).

The "assessing heart failure therapy" as used herein, preferably refers to the identification of patients who are eligible to change of heart failure therapy as described elsewhere herein. Thus, patients can be identified or selected which would benefit from a change of therapy.

Thus, the present invention relates to a method of identifying a patient who is eligible to an adapted heart failure therapy, said method comprising the steps of
a) determining the amount of a BNP-type peptide, such as NT-proBNP, in a baseline sample from a patient who has been hospitalized for acute heart failure, wherein said baseline sample has been obtained prior to discharge of the patient from hospital,
b) determining the amount of the BNP-type peptide in at least one further sample wherein said at least one further sample has been obtained after discharge of the patient, and
c) comparing the amount in the baseline sample determined in step a) to the amount in the at least one further sample determined in step b), and
d) identifying a patient who is eligible to an adapted heart failure therapy.

The phrase "assessing heart failure therapy", "selecting a patient" or "identifying a patient" as used herein refers to using the information or data generated relating to the level of the at least one marker as referred to in the context of the present invention in a sample of a patient to identify or selecting the patient as more likely to benefit or less likely to benefit from an adaptation of the therapy as referred to herein.

As will be understood by those skilled in the art, the assessment made by the method of the present invention is usually not intended to be correct for 100% of the subjects to be diagnosed. The term, however, typically requires that the assessment is correct for a statistically significant portion of the subjects (e.g. a cohort in a cohort study). Whether a portion is statistically significant can be determined without further ado by the person skilled in the art using various well known statistic evaluation tools, e.g., determination of confidence intervals, p-value determination, Student's t-test, Mann-Whitney test etc.. Details are found in Dowdy and Wearden, Statistics for Research, John Wiley & Sons, New York 1983. Pre-ferred confidence intervals are at least 90%, at least 95%, at least 97%, at least 98% or at least 99 %. The p-values are, preferably, 0.1, 0.05, 0.01, 0.005, or 0.0001.

It is to be understood that a subject who is eligible to an adapted/change therapy as referred to herein, typically will benefit from the therapy. In particular, a subject benefits from the therapy, if the therapy reduces the blood level of NT-proBNP (or BNP) in said patient. Alternatively, the therapy reduces the risk of readmission to hospital due to heart failure of said subject and/or of death, in particular within about 180 days after discharge. Also, the therapy may lead to an improvement in congestion and quality of life.

The terms "subject" and "patient" are used interchangeably herein. The "patient" as referred to herein is, preferably, a mammal. Mammals include, but are not limited to, domesticated animals (e.g., cows, sheep, cats, dogs, and horses), primates (e.g., humans and non-human primates such as monkeys), rabbits, and rodents (e.g., mice and rats). In certain embodiments, the patient is a human patient.

The patient to be tested shall have been hospitalized for acute heart failure (AHF). The term "acute heart failure" is well known in the art and e.g. described in the 2021 ESC guidelines (Authors/Task Force Members, McDonagh et al., Reference 17). Typically, the term AHF refers to rapid or gradual onset of symptoms and/or signs of HF, severe enough for the patient to seek urgent medical attention leading to an unplanned hospital admission. Patients with AHF require urgent evaluation AHF is a leading cause of hospitalizations in subjects aged >65 years and is associated with high mortality and rehospitalization rates.

In an embodiment, the patient has a reduced left ventricular ejection fraction.

In alternative embodiment, the patient has preserved left ventricular rejection fraction.

The present invention deals with the adaptation of oral heart failure therapies

The patient to be tested, typically, is already treated for heart failure at the time of the discharge from hospital (preferably with heart failure medicaments, in particular oral heart failure medicaments). Thus, the patient already receives oral medical heart failure therapy. Preferably, the patient receives at least one diuretic and one or more components (medicaments), such as two components or three components of Guideline-Directed Medical Therapy (GDMT), in particular Guideline-Directed Medical Therapy as disclosed in the 2021 ESC Guidelines (Reference 17, herewith incorporated by reference).

Preferably, the guideline directed medical therapy comprises the administration of one or more of the following (oral) medicaments
i. at least one beta-blocker (BB);
ii. at least one angiotensin converting enzyme inhibitor (ACEi), at least one angiotensin receptor blocker (ARB), and/or at least one angiotensin receptor neprilysin inhibitor (ARNi),
iii. at least one mineralocorticoid receptor antagonist (MRA), and/or
iv. at least one SGLT2 inhibitor.

Preferably, the guideline directed medical therapy comprises the administration of at least one medicament all of the three medicaments classes i, ii and iii, i.e. of i. at least one beta-blocker (BB); ii. at least one angiotensin converting enzyme inhibitor (ACEi), at least one angiotensin receptor blocker (ARB), and at least one angiotensin receptor neprilysin inhibitor (ARNi), and iii. at least one mineralocorticoid receptor antagonist (MRA).

Preferred BBs, ACEis, ARBs, ARNis and MRAs are disclosed in Table A. Further, the table contains information on the optimal dosage of the individual medicaments (BBs, ACEis, ARBs, ARNis and MRAs).

In an embodiment, the medicament is a beta blocker. For example, the beta blocker is bisoprolol.

In an embodiment, the medicament is an angiotensin converting enzyme inhibitor. For example, the angiotensin converting enzyme inhibitor is Captopril.

In an embodiment, the medicament is an angiotensin receptor blocker. For example, the angiotensin receptor blocker is Lorsatan. Alternatively, the angiotensin receptor blocker is Lorsatan.

In an embodiment, the medicament is an angiotensin receptor neprilysin inhibitor. For example, the angiotensin receptor neprilysin inhibitor is Sacubitril/valsartan.

In an embodiment, the medicament is a mineralocorticoid receptor antagonist. For example, the mineralocorticoid receptor antagonist is Spironolactone. Alternatively, the mineralocorticoid receptor antagonist is Eplerenone.

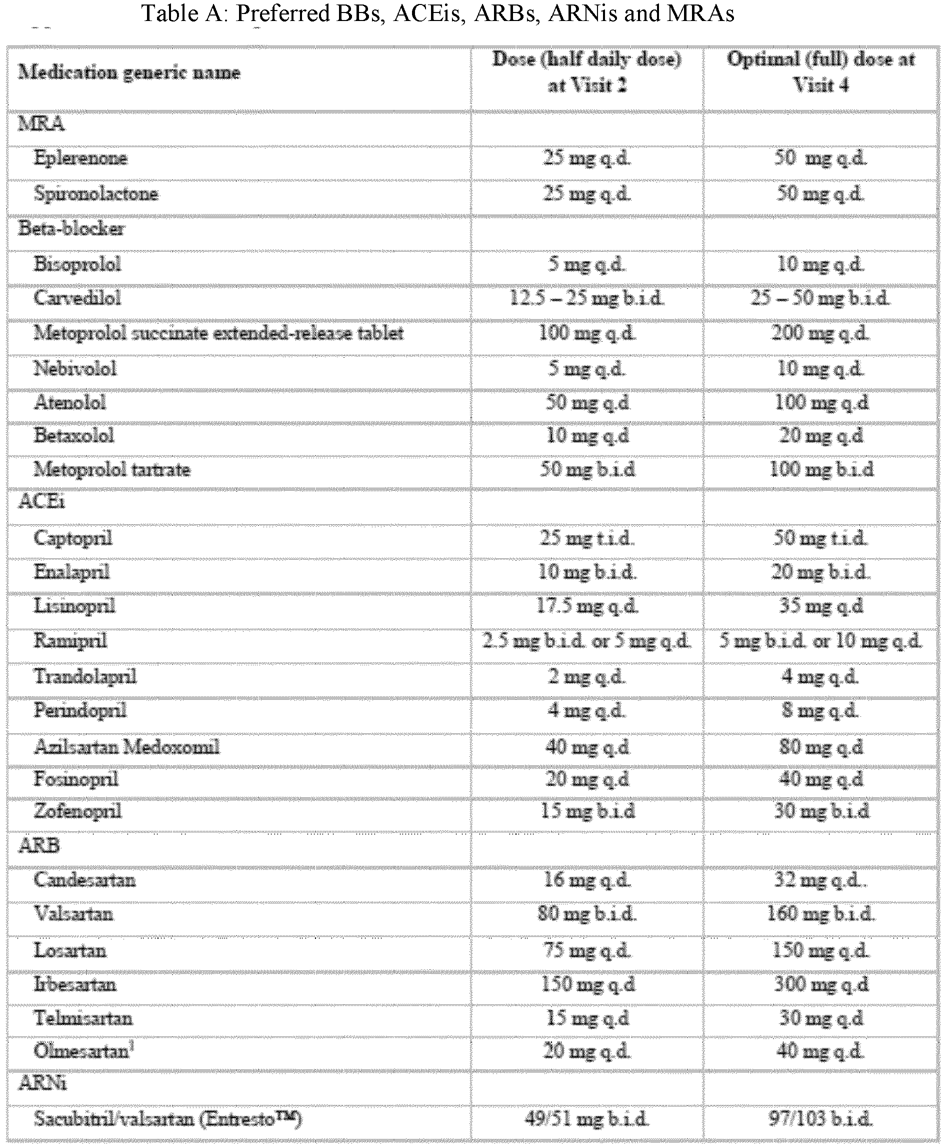

At discharge, the patient preferably is administered a medical therapy with the individual medicaments, preferably at at least half of the optimal dose. In some embodiments, the dose is increased later to the optimal dose.

Preferably, the patient has not yet reached the target dose for the one or more components of GMDT at the time point at which the at least one further sample is obtained. Thus, the current dose of the one or more components is lower that the target dose.

In addition to the guideline directed medical therapy, the patient preferably is treated with at least one diuretic (at discharge and when the at least one further sample is obtained). Thus, the patient is treated with one or more components of GMDT (see i to iv above) and at least one diuretic.

A diuretic is any substance that promotes diuresis, the increased production of urine. Thus, they increase the elimination of sodium and water via urine.

Diuretics are diuretics that increase the elimination of sodium and water via urine. A particularly preferred diuretic is a loop diuretic or a thiazide diuretic.

Preferably, loop diuretics are used due to their efficacy and rapid onset of action. They act on the ascending loop of Henle in the kidney. Preferably, the loop diuretic is selected from the group of furosemide, azosemide, bumetanide, piretanide, torasemide, ethacrynic acid, etozolin. In particular, the loop diuretic is furosemide. Atypical dose is 20 to 80 mg per day. Alternatively, the loop diuretic is torasemide. A typical dose is 10 to 20 mg per day.

The methods of the present invention encompass the determination of the amount of a BNP-type peptide in a baseline sample and in at least one further sample.

The term "sample" as used herein refers to any sample that under physiological conditions comprises the biomarkers referred to herein. More typically, the sample is a body fluid sample, e.g. a blood sample or sample derived therefrom, a urine sample, a saliva sample a lymphatic fluid sample or the like. Most typically, said sample is a blood sample or a sample derived therefrom. Accordingly, the sample may be a blood, serum or plasma sample. Blood samples, typically, include capillary, venous or arterial blood samples. In an embodiment, the sample is an interstitial fluid sample.

The "baseline sample" is typically obtained from the patient prior to discharge from hospital. In an embodiment, the baseline sample has been obtained from the patient within two days prior to the discharge of the patient from the hospital.

The amount of the biomarker as referred to herein shall be also determined in at least one further sample that has been obtained from the patient after discharge of the patient. Typically, said at least one further sample is obtained within 8 weeks after discharge, such as within six weeks, or within four weeks after discharge. Typically, said at least one further sample is obtained within 8 weeks after the baseline sample, such as within six weeks, or within four weeks after the baseline sample.

Also typically, the at least one further sample has been obtained from the patient about 1, about 2, about 3, and/or about 6 weeks after discharge from hospital. Also typically, the at least one further sample has been obtained from the patient about 1, about 2, about 4, and/or about 6 weeks after discharge from hospital.

In a preferred embodiment, at least two further samples are obtained, e.g. in samples obtained about 1 week and about 2 weeks after discharge (or the baseline sample). In another preferred embodiment, at least three further samples are obtained, e.g. in samples obtained about 1 week, about 2 weeks and about 3 weeks after discharge (or the baseline sample). In another preferred embodiment, at least four further samples are obtained, e.g. in samples obtained about 1 week, about 2 weeks, about 3 weeks and about 6 weeks after discharge (or the baseline sample). In yet another preferred embodiment, at least four further samples are obtained, e.g. in samples obtained about 1 week, about 2 weeks, about 4 weeks and about 6 weeks after discharge (or the baseline sample).

As used herein, the term "BNP-type peptides" comprise pre-proBNP, proBNP, NT-proBNP, and BNP. The pre-pro peptide (134 amino acids in the case of pre-proBNP) comprises a short signal peptide, which is enzymatically cleaved off to release the pro peptide (108 amino acids in the case of proBNP). The pro peptide is further cleaved into an N-terminal pro peptide (NT-pro peptide, 76 amino acids in case of NT-proBNP) and the active hormone (32 amino acids in the case of BNP). Preferably, BNP-type peptides according to the present invention are NT-proBNP, BNP, and variants thereof. BNP is the active hormone and has a shorter half-life than the respective inactive counterpart NT-proBNP. BNP is metabolized in the blood, whereas NT-proBNP circulates in the blood as an intact molecule and as such is eliminated renally. The in-vivo half-life of NT-proBNP is 120 min longer than that of BNP, which is 20 min (Smith 2000, J Endocrinol. 167: 239-46.). Preanalytics are more robust with NT-proBNP allowing easy transportation of the sample to a central laboratory (Mueller 2004, Clin Chem Lab Med 42: 942-4.). Blood samples can be stored at room temperature for several days or may be mailed or shipped without recovery loss. In contrast, storage of BNP for 48 hours at room temperature or at 4° Celsius leads to a concentration loss of at least 20 % (Mueller loc.cit.; Wu 2004, Clin Chem 50: 867-73.). Therefore, depending on the time-course or properties of interest, either measure-ment of the active or the inactive forms of the natriuretic peptide can be advantageous. The most preferred natriuretic peptides according to the present invention are NT-proBNP or variants thereof. As briefly discussed above, the human NT-proBNP, as referred to in accordance with the present invention, is a polypeptide comprising, preferably, 76 amino acids in length corre-sponding to the N-terminal portion of the human NT-proBNP molecule. The structure of the human BNP and NT-proBNP has been described already in detail in the prior art, e.g., WO 02/089657, WO 02/083913 or Bonow loc. cit. Preferably, human NT-proBNP as used herein is human NT-proBNP as disclosed in EP 0 648 228 B1. These prior art documents are herewith incorporated by reference with respect to the specific sequences of NT-proBNP and variants thereof disclosed therein.

In an embodiment, the BNP-type peptide is NT-proBNP.

In another embodiment, the BNP-type peptide is BNP.

The term "level" or "amount" as used herein encompasses the absolute amount of a biomarker as referred to herein, the relative amount or concentration of the said biomarker as well as any value or parameter which correlates thereto or can be derived therefrom. Such values or parameters comprise intensity signal values from all specific physical or chemical properties obtained from the said peptides by direct measurements, e.g., intensity values in mass spectra or NMR spectra. Moreover, encompassed are all values or parameters which are obtained by indirect measurements specified elsewhere in this description, e.g., response amounts determined from biological read out systems in response to the peptides or intensity signals obtained from specifically bound ligands. It is to be understood that values correlating to the aforementioned amounts or parameters can also be obtained by all standard mathematical operations.

In an embodiment, the term "amount" refers to the concentration, for example the mass concentration (mass of a biomarker divided by the volume of the sample, such as the blood, serum or plasma sample). For example, the concentration of the biomarker in the sample can be given as "pg/ml".

The biomarkers as referred to herein can be detected using methods generally known in the art. Methods of detection generally encompass methods to quantify the level of a biomarker in the sample (quantitative method). It is generally known to the skilled artisan which of the following methods are suitable for qualitative and/or for quantitative detection of a biomarker. Samples can be conveniently assayed for, e.g., proteins using Westerns and immunoassays, like ELISAs, RIAs, fluorescence-based immunoassays, which are commercially available. Further suitable methods to detect biomarker include measuring a physical or chemical property specific for the peptide or polypeptide such as its precise molecular mass or NMR spectrum. Said methods comprise, e.g., biosensors, optical devices coupled to immunoassays, biochips, analytical devices such as mass- spectrometers, NMR-analyzers, or chromatography devices. Further, methods include microplate ELISA-based methods, fully-automated or robotic immunoassays (available for example on Elecsys^{™} analyzers), CBA (an enzymatic Cobalt Binding Assay, available for example on Roche-Hitachi^{™} analyzers), and latex agglutination assays (available for example on Roche-Hitachi^{™} analyzers).

For the detection of biomarker proteins as referred to herein a wide range of immunoassay techniques using such an assay format are available, see, e.g., U.S. Pat. Nos. 4,016,043, 4,424,279, and 4,018,653. These include both single-site and two-site or "sandwich" assays of the non-competitive types, as well as in the traditional competitive binding assays. These assays also include direct binding of a labeled antibody to a target biomarker.

Sandwich assays are among the most useful and commonly used immunoassays.

Methods for measuring electrochemiluminescent phenomena are well-known. Such methods make use of the ability of special metal complexes to achieve, by means of oxidation, an excited state from which they decay to ground state, emitting electrochemiluminescence. For review see Richter, M.M., Chem. Rev. 104 (2004) 3003-3036.

Biomarkers can also be detected by generally known methods including magnetic resonance spectroscopy (NMR spectroscopy), Gas chromatography-mass spectrometry (GC-MS), Liquid chromatography-mass spectrometry (LC-MS), High and ultra-HPLC HPLC such as reverse phase HPLC, for example, ion-pairing HPLC with dual UV-wavelength detection, capillary electrophoresis with laser-induced fluorescence detection, anion exchange chromatography and fluorescent detection, thin layer chromatography.

Preferably, measuring the level of a biomarker as referred to herein comprises the steps of (a) contacting a cell capable of eliciting a cellular response the intensity of which is indicative of the level of the peptide or polypeptide with the said peptide or polypeptide for an adequate period of time, (b) measuring the cellular response. For measuring cellular responses, the sample or processed sample is, preferably, added to a cell culture and an internal or external cellular response is measured. The cellular response may include the measurable expression of a reporter gene or the secretion of a substance, e.g. a peptide, polypeptide, or a small molecule. The expression or substance shall generate an intensity signal which correlates to the level of the peptide or polypeptide.

Also preferably, measuring the level of a peptide or polypeptide comprises the step of measuring a specific intensity signal obtainable from the peptide or polypeptide in the sample. As described above, such a signal may be the signal intensity observed at an m/z variable specific for the peptide or polypeptide observed in mass spectra or a NMR spectrum specific for the peptide or polypeptide.

Measuring the level of a peptide or polypeptide may, preferably, comprises the steps of (a) contacting the peptide with a specific binding agent, (b) (optionally) removing non-bound binding agent, (c) measuring the level of bound binding agent, i.e. the complex of the binding agent formed in step(a). According to a preferred embodiment, said steps of contacting, removing and measuring may be performed by an analyzer unit of the system disclosed herein. According to some embodiments, said steps may be performed by a single analyzer unit of said system or by more than one analyzer unit in operable communication with each other. For example, according to a specific embodiment, said system disclosed herein may include a first analyzer unit for performing said steps of contacting and removing and a second analyzer unit, operably connected to said first analyzer unit by a transport unit (for example, a robotic arm), which performs said step of measuring.

The bound binding agent, i.e. the binding agent or the binding agent/peptide complex, will generate an intensity signal. Binding according to the present invention includes both covalent and non-covalent binding. A binding agent according to the present invention can be any compound, e.g., a peptide, polypeptide, nucleic acid, or small molecule, binding to the peptide or polypeptide described herein. Preferred binding agents include antibodies, nucleic acids, peptides or polypeptides such as receptors or binding partners for the peptide or polypeptide and fragments thereof comprising the binding domains for the peptides, and aptamers, e.g. nucleic acid or peptide aptamers. Methods to prepare such binding agents are well-known in the art. For example, identification and production of suitable antibodies or aptamers is also offered by commercial suppliers. The person skilled in the art is familiar with methods to develop derivatives of such binding agents with higher affinity or specificity. For example, random mutations can be introduced into the nucleic acids, peptides or polypeptides. These derivatives can then be tested for binding according to screening procedures known in the art, e.g. phage display. Antibodies as referred to herein include both polyclonal and monoclonal antibodies, as well as fragments thereof, such as Fv, Fab and F(ab)2 fragments that are capable of binding the antigen. The present invention also includes single chain antibodies and humanized hybrid antibodies wherein amino acid sequences of a non-human donor antibody exhibiting a desired antigen-specificity are combined with sequences of a human acceptor antibody. The donor sequences will usually include at least the antigen-binding amino acid residues of the donor but may comprise other structurally and/or functionally relevant amino acid residues of the donor antibody as well. Such hybrids can be prepared by several methods well known in the art. Preferably, the binding agent or agent binds specifically to the pep-tide or polypeptide. Specific binding according to the present invention means that the ligand or agent should not bind substantially to ("cross-react" with) another peptide, polypeptide or substance present in the sample to be analyzed. Preferably, the specifically bound peptide or polypeptide should be bound with at least 3 times higher, more preferably at least 10 times higher and even more preferably at least 50 times higher affinity than any other relevant peptide or polypeptide. Non-specific binding may be tolerable, if it can still be distinguished and measured unequivocally, e.g. according to its size on a Western Blot, or by its relatively higher abundance in the sample. Binding of the binding agent can be measured by any method known in the art. Preferably, said method is semi-quantitative or quantitative. Further suitable techniques for the determination of a polypeptide or peptide are described in the following.

Binding of a binding agent may be measured directly, e.g. by NMR or surface plasmon resonance. Measurement of the binding of a binding agent, according to preferred embodiments, is performed by an analyzer unit of a system disclosed herein. Thereafter, a level of the measured binding may be calculated by a computing device of a system disclosed herein. If the binding agent also serves as a substrate of an enzymatic activity of the pep-tide or polypeptide of interest, an enzymatic reaction product may be measured (e.g. the level of a protease can be measured by measuring the level of cleaved substrate, e.g. on a Western Blot). Alternatively, the binding agent may exhibit enzymatic properties itself and the "binding agent/peptide or polypeptide" complex or the binding agent which was bound by the peptide or polypeptide, respectively, may be contacted with a suitable substrate allowing detection by the generation of an intensity signal. For measurement of enzymatic reaction products, preferably the level of substrate is saturating. The substrate may also be labeled with a detectable label prior to the reaction. Preferably, the sample is contacted with the substrate for an adequate period of time. An adequate period of time refers to the time necessary for a detectable, preferably measurable, level of product to be produced. Instead of measuring the level of product, the time necessary for appearance of a given (e.g. detectable) level of product can be measured. Third, the binding agent may be coupled covalently or non-covalently to a label allowing detection and measurement of the binding agent. Labeling may be done by direct or indirect methods. Direct labeling involves coupling of the label directly (covalently or non-covalently) to the binding agent. Indirect labeling involves binding (covalently or non-covalently) of a secondary binding agent to the first binding agent. The secondary binding agent should specifically bind to the first binding agent. Said secondary binding agent may be coupled with a suitable label and/or be the target (receptor) of tertiary binding agent binding to the secondary binding agent. The use of secondary, tertiary or even higher order binding agents is often used to increase the signal. Suitable secondary and higher order binding agents may include antibodies, secondary antibodies, and the well-known streptavidin-biotin system (Vector Laboratories, Inc.). The binding agent or substrate may also be "tagged" with one or more tags as known in the art. Such tags may then be targets for higher order binding agents. Suitable tags include biotin, digoxygenin, His-Tag, Glutathion-S-Transferase, FLAG, GFP, myc-tag, influenza A virus haemagglutinin (HA), maltose binding protein, and the like. In the case of a peptide or polypeptide, the tag is preferably at the N-terminus and/or C-terminus. Suitable labels are any labels detectable by an appropriate detection method. Typical labels include gold particles, latex beads, acridan ester, luminol, ruthenium, enzymatically active labels, radioactive labels, magnetic labels ("e.g. magnetic beads", including paramagnetic and superparamagnetic labels), and fluo-rescent labels. Enzymatically active labels include e.g. horseradish peroxidase, alkaline phosphatase, beta-Galactosidase, Luciferase, and derivatives thereof. Suitable substrates for detection include di-amino-benzidine (DAB), 3,3'-5,5'-tetramethylbenzidine, NBT-BCIP (4-nitro blue tetrazolium chloride and 5-bromo-4-chloro-3-indolyl-phosphate, avail-able as ready-made stock solution from Roche Diagnostics), CDP-Star^{™} (Amersham Bio-sciences), ECF^{™} (Amersham Biosciences). A suitable enzyme-substrate combination may result in a colored reaction product, fluorescence or chemoluminescence, which can be measured according to methods known in the art (e.g. using a light-sensitive film or a suit-able camera system). As for measuring the enzymatic reaction, the criteria given above apply analogously. Typical fluorescent labels include fluorescent proteins (such as GFP and its derivatives), Cy3, Cy5, Texas Red, Fluorescein, and the Alexa dyes (e.g. Alexa 568). Further fluorescent labels are available e.g. from Molecular Probes (Oregon). Also the use of quantum dots as fluorescent labels is contemplated. A radioactive label can be detected by any method known and appropriate, e.g. a light-sensitive film or a phosphor imager.

The level of a peptide or polypeptide may be, also preferably, determined as follows: (a) contacting a solid support comprising a binding agent for the peptide or polypeptide as specified above with a sample comprising the peptide or polypeptide and (b) measuring the level peptide or polypeptide which is bound to the support. The binding agent, preferably chosen from the group consisting of nucleic acids, peptides, polypeptides, antibodies and aptamers, is preferably present on a solid support in immobilized form. Materials for manufacturing solid supports are well known in the art and include, inter alia, commercially available column materials, polystyrene beads, latex beads, magnetic beads, colloid metal particles, glass and/or silicon chips and surfaces, nitrocellulose strips, membranes, sheets, duracytes, wells and walls of reaction trays, plastic tubes etc. The binding agent or agent may be bound to many different carriers. Examples of well-known carriers include glass, polystyrene, polyvinyl chloride, polypropylene, polyethylene, polycarbonate, dextran, nylon, amyloses, natural and modified celluloses, polyacrylamides, agaroses, and magnetite. The nature of the carrier can be either soluble or insoluble for the purposes of the invention. Suitable methods for fixing/immobilizing said binding agent are well known and include, but are not limited to ionic, hydrophobic, covalent interactions and the like. It is also contemplated to use "suspension arrays" as arrays according to the present invention (No-lan 2002, Trends Biotechnol. 20(1):9-12). In such suspension arrays, the carrier, e.g. a microbead or microsphere, is present in suspension. The array consists of different microbeads or microspheres, possibly labeled, carrying different binding agents. Methods of producing such arrays, for example based on solid-phase chemistry and photo-labile protective groups, are generally known (US 5,744,305).

The term "detection agent" as used herein refers to an agent that is capable of secifically recognizing and binding to the biomarker present in a sample. The term is used interchangeably with the term "ligand" herein. Moreover, the said agent shall allow for direct or indirect detection of the complex formed by the said agent and the biomarker. Direct detection can be achieved by including into the agent a detectable label. Indirect labeling maybe achieved by a further agent that specifically binds to the complex comprising the biomarker and the detection agent wherein the said further agent is than capable of generating a detectable signal. Suitable compounds which can be used as detection agents are well known in the art. Preferably, the detection agent is an antibody, in particular a monoclonal antibody, or aptamere which specifically binds to the biomarker as referred to herein. The term "antibody" herein is used in the broadest sense and encompasses various anti-body structures, including but not limited to monoclonal antibodies, polyclonal antibodies, multispecific antibodies (e.g., bispecific antibodies), and antibody fragments so long as they exhibit the desired antigen-binding activity. Preferably, the antibody is a polyclonal antibody. More preferably, the antibody is a monoclonal antibody.

The term "comparing" as used herein, typically, refers to comparing the level or amount of the biomarker in the at least one further sample with the level of the biomarker in the baseline sample. It is to be understood that comparing as used herein usually refers to a comparison of corresponding parameters or values, e.g., an absolute amount is compared to an absolute reference amount while a concentration is compared to a reference concentration or an intensity signal obtained from the biomarker in a sample is compared to the same type of intensity signal obtained from the baseline sample. The comparison may be carried out manually or computer assisted. Thus, the comparison may be carried out by a computing device (e.g., of a system disclosed herein). The value of the measured or detected level of the biomarker in the sample from the individual or patient and the reference level can be, e.g., compared to each other and the said comparison can be automatically carried out by a computer program executing an algorithm for the comparison. The computer program carrying out the said evaluation will provide the desired assessment in a suitable output format. The computer program may further evaluate the result of the comparison, i.e. automatically provides the desired assessment in a suitable output format.

Based on the comparison step carried out in the context of the method of the present invention, the heart failure therapy can be assessed. In particular, a patient can be identified who requires or is eligible to an adaption of the heart failure therapy.

Preferably, an increase of the amount of the BNP-type peptide in the at least one further sample as compared to the amount in the baseline sample is indicative for a patient who is eligible to an adaption of the heart failure therapy. More preferably, an increase of the amount of the BNP-type peptide in the at least one further sample of at least 10%, such as at least 20%, at least 30%, at least 40% or at least 50% as compared to the amount in the baseline sample is indicative for a patient who is eligible to an adaption of the heart failure therapy.

If the amount of the BNP-type peptide is increased, the therapy is preferably adapted as follows: an increase of the dose of at the least one diuretic (in at least one of the further samples), but no increase of the dose of one or more components of guideline directed medical therapy (GDMT). Thus, the dose of at the least one diuretic, but the dose of guideline directed medical therapy (GDMT) is not increased, i.e. the dose is maintained or reduced (preferably reduced).

An increase of the dose of at the least one diuretic is preferably an increase of the dose of at least 20%, such as at least 30% or at least 50%, or least 100% (as compared the current dose).

Also, the increase may be an increase of between 20% to 100%, such as between 50% to 100%.

No increase of the dose of guideline directed medical therapy means that the dose(s) of the individual medicament(s) of the GDMT is (are) maintained or reduced. A reduction, typically, is a reduction of up to 50%, such as up to 40%, such as 30% of the current dose(s). For example, the reduction typically is a reduction of between 20% to 50% of the current dose.

In case the therapy is adapted, the patient might be subjected to an additional safety visit after 1 week, inclusive of NTproBNP measurement.

Further, it is envisaged that further parameters are assessed at the individual visits of the patient, in particular the parameters as described in Ref. 18 (Kimmoun et al.) or Ref. 19 (Cotter et al.).

These parameters are one or more of the following parameters
- the patient's systolic blood pressure
- the patient's blood, serum or plasma potassium (K) level, in particular the serum potassium level
- clinical signs of congestion
- the patient's eGFR
- the patient's heart rate

ACEi/ARB/ARNi and/or MRAs are not to be up-titrated immediately if the systolic blood pressure is < 95 mmHg, the serum potassium > 5.0 mmol/L or the eGFR is < 30 ml/min/1.73m2. Beta-blockers are not to be up-titrated if heart rate < 55 bpm or systolic BP is < 95 mmHg. If the NT-proBNP level was >10% higher than the pre-discharge level, physicians should have considered not up-titrating beta-blockers and considered increasing diuretics. In case, up-titration to full optimal doses of one or all of the medications (beta-blockers, ACEi/ARB/ARNi, and a MRA) is not achieved, additional visit(s) are scheduled to implement full optimal doses.

The definitions and explanations provided above preferably apply mutatis mutandis to the following.

The methods of the present invention can be also carried out as computer-implemented methods. In computer-implemented methods, typically, all steps of the computer-implemented method of the present invention are performed by one or more processing units of a computer or a computer network. However, the computer-implemented method may comprise additional steps, such as the determination of the amount of a marker in a sample, such as the amount of a BNP-type peptide.

Thus, the present invention further relates to a computer-implemented method for assessing heart failure therapy in a patient who has been hospitalized for acute heart failure, said method comprising the steps of
a) receiving, at a processing unit, a value for the amount of a BNP-type peptide in a baseline sample, wherein said baseline sample has been obtained prior to discharge of the patient from hospital,
b) receiving, at the processing unit, a value determining the amount of the BNP-type peptide in at least one further sample wherein said at least one further sample has been obtained after discharge of the patient, and
c) carrying out at the processing unit a comparison of the amount in the baseline sample determined in step a) to the amount in the at least one further sample determined in step b), and
wherein an increase, an unchanged amount, or a decrease of less than 10% of the amount of the BNP-type peptide in the at least one further sample as compared to the amount in the baseline sample is indicative for a patient who is eligible to adapted heart failure therapy, such as an increase of the dose of at least one diuretic, but not to an increase of the dose of guideline directed medical therapy (GDMT).

In a further aspect, the present invention relates to a method of treating a patient who has been hospitalized for acute heart failure, said method comprising the steps of
a) determining the amount of a BNP-type peptide, such as NT-proBNP, in a baseline sample, wherein said baseline sample has been obtained prior to discharge of the patient from hospital,
b) determining the amount of the BNP-type peptide in at least one further sample wherein said at least one further sample has been obtained after discharge of the patient,
c) comparing the amount in the baseline sample determined in step a) to the amount in the at least one further sample determined in step b),
d) identifying a patient as being eligible to adapted heart failure therapy, such as an increase of the dose of at least one diuretic, but not to an increase of the dosage of one or more components of guideline directed medical therapy (GDMT), preferably based the results of step c),
e) treating the identified patient with an increased dose of at least one diuretic, while maintaining the dose of guideline directed medical therapy (GDMT).

The expression that dosage of one or more components of guideline directed medical therapy is not increased, typically means that the dosage is not increased immediately. Typically, the increase of the dosage is delayed. However, at later visits the dose could be increased, for example, if the amount of the biomarker is decreased by at more than 10%.

The present invention further relates to the *in vitro* use of
i. at least one BNP-type peptide or
ii. at least one detection agent for the at least one BNP-type peptide
in a baseline sample and in at least one further sample obtained from a patient who has been hospitalized for acute heart failure for assessing heart failure therapy in said patient,
wherein said baseline sample has been obtained prior to discharge of the patient from hospital, and said at least one further sample has been obtained after discharge of the patient.

The present invention further relates to a computer program including computer-executable instructions for performing the steps of the computer-implemented method according to the present invention, when the program is executed on a computer or computer network. Typically, the computer program specifically may contain computer-executable instructions for performing the steps of the method as disclosed herein. Specifically, the computer program may be stored on a computer-readable data carrier.

The present invention further relates to a computer program product with program code means stored on a machine-readable carrier, in order to perform the method according to the present invention, when the program is executed on a computer or computer network, such as one or more of the above-mentioned steps discussed in the context of the computer program. As used herein, a computer program product refers to the program as a tradable product. The product may generally exist in an arbitrary format, such as in a paper format, or on a computer-readable data carrier. Specifically, the computer program product may be distributed over a data network.

The present invention further relates to a computer or computer network comprising at least one processing unit, wherein the processing unit is adapted to perform all steps of the method according to the present invention, in particular steps a), b), c) and d).

The present invention also, in principle, contemplates a computer program, computer program product or computer readable storage medium having tangibly embedded said computer program, wherein the computer program comprises instructions when run on a data processing device or computer carry out the method of the present invention as specified above. Specifically, the present disclosure further encompasses:
- A computer or computer network comprising at least one processor, wherein the processor is adapted to perform the method according to one of the embodiments described in this description,
- a computer loadable data structure that is adapted to perform the method according to one of the embodiments described in this description while the data structure is being executed on a computer,
- a computer script, wherein the computer program is adapted to perform the method according to one of the embodiments described in this description while the program is being executed on a computer,
- a computer program comprising program means for performing the method according to one of the embodiments described in this description while the computer program is being executed on a computer or on a computer network,
- a computer program comprising program means according to the preceding embodiment, wherein the program means are stored on a storage medium readable to a computer,
- a storage medium, wherein a data structure is stored on the storage medium and wherein the data structure is adapted to perform the method according to one of the embodiments described in this description after having been loaded into a main and/or working storage of a computer or of a computer network,
- a computer program product having program code means, wherein the program code means can be stored or are stored on a storage medium, for performing the method according to one of the embodiments described in this description, if the program code means are executed on a computer or on a computer network,
- a data stream signal, typically encrypted, comprising a data for the BNP-type peptide, and
- a data stream signal, typically encrypted, comprising the calculations by the methods of the present invention and, preferably, providing information of the assessment.

The present invention, in particular, relates to a device for assessing heart failure therapy, said device comprising a processing unit, and a computer program including computer-executable instructions, wherein said instructions, when executed by the processing unit, cause the processing unit to perform the computer-implemented method of the present invention.

For example, the present invention relates to a device for assessing heart failure therapy, said device comprising a processing unit, and a computer program including computer-executable instructions (such as a computer program as set forth above), wherein said instructions, when executed by the processing unit, cause the processing unit to perform the computer-implemented method according to the present invention, i.e. to perform the steps of said method. The device may further comprise a user interface and a display, wherein the processing unit is coupled to the user interface and the display. Typically, the device provides as output the assessment. In an embodiment, the assessment is provided on the display. Typically, the device comprises software being tangibly embedded into said device and, when running on said device, carries out the method of the present invention.

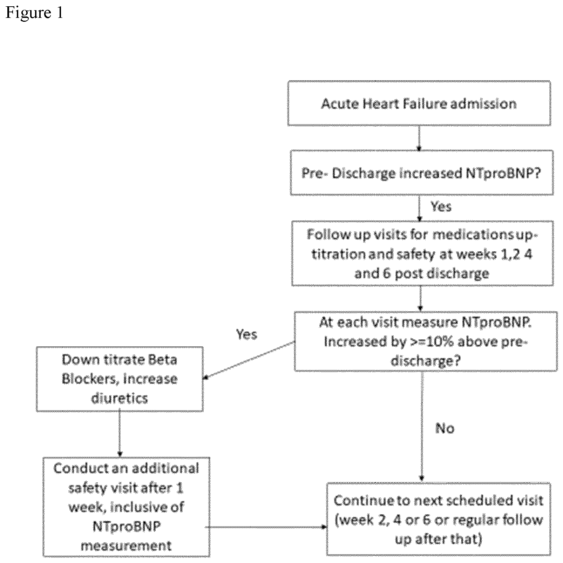

**Figure 1** shows an exemplary depiction of the method of the present invention.

All patents, patent applications, and publications or public disclosures referred to or cited herein are incorporated by reference in their entirety.

The invention will be further described with reference to the examples described herein; however, it is to be understood that the invention is not limited to such examples.

### Examples

Patients 1, 2 and 3 were patients in the STRONG-HF study. The study design was previously published (18,19). In short, this was a multicenter, randomized, parallel-group study designed to evaluate the efficacy and safety of up-titration of standard of care medical therapy including beta-blockers, ACEi (or ARB if intolerant to ACEi) or ARNi and MRAs, on morbidity and mortality when initiated and up-titrated early during hospitalization for acute heart failure (AHF). Eighteen hundred (1800) patients were planned to be enrolled in the study. The protocol was amended twice: first primarily to add a patient contact at 180 days for safety and, second, in order to increase study power, to increase target enrollment from 900 to 1800 and to change the primary endpoint from 90 to 180 days. Patients with AHF with clinical signs of congestion and elevated circulating NT-proBNP and who were not treated with optimal doses of oral HF therapies within 2 days before hospital discharge for AHF and who were hemodynamically stable were randomized 1:1 to usual care ("Usual Care", UC) or intensification of treatment with beta-blockers, and ACEi (or ARB) or ARNi, and a MRA ("High Intensity Care", HIC). Randomization was stratified by left ventricular ejection fraction (LVEF ≤ 40 versus >40%) and country with blocks of size 30 within country and randomly-ordered sub-blocks of size 2, 4 and 6 and employed a central interactive web randomization system. Patients randomized to the HIC arm followed an algorithm combining optimization of oral HF therapies and frequent visits, including circulating NT-proBNP measures, to assess congestion. The study was approved by appropriate competent authorities and ethics committees, and patients provided written informed consent. The first dose adjustment occurred just following randomization (Visit 2, within 2 days before discharge), when patients were prescribed medical therapy with beta-blockers, ACEi (or ARB if intolerant to ACEi) or ARNi and MRA adjusted to at least half the optimal doses. Patients were assessed by the study team at 1, 2, 3, and 6 weeks (Visits 3, 4, 5 and 6) following randomization. At Visit 4 (2 weeks following randomization) up-titration to full optimal doses of beta-blockers, ACEi/ARB/ARNi, and a MRA should have been performed given adequate safety. A safety visit was performed 1 week after any up-titration in cases where up-titration had to be delayed. Safety and tolerability were assessed at Visits 3 to 6 by full physical examination, and laboratory assessments of NT-proBNP, sodium, potassium, glucose, and kidney function and hemoglobin measures. Investigators assessed congestion, and increased diuretics as needed, based on those assessments. ACEi/ARB/ARNi and/or MRAs were not to be up-titrated if the systolic blood pressure was < 95 mmHg, serum potassium > 5.0 mmol/L or eGFR was < 30 ml/min/1.73m2. Beta-blockers were not to be up-titrated if heart rate < 55 bpm or systolic BP was < 95 mmHg. If the NT-proBNP level was >10% higher than the pre-discharge level, physicians should have considered not up-titrating beta-blockers and considered increasing diuretics. In case, up-titration to full optimal doses of one or all of the medications (beta-blockers, ACEi/ARB/ARNi, and a MRA) was not achieved, additional visit(s) were scheduled to implement full optimal doses. All randomized patients, and screen failures, were followed at 90 days (Visit 7) for the occurrence of hospital re-admissions or death. Adverse events and outpatient visits were collected through 90 days for all randomized patients. Events were reported as they occurred throughout the follow-up period.

Thus, the patients were assessed at different visits.
V2: Visit 2, within 2 days before discharge
V3, V4, V5 and V6: at 1, 2, 3, and 6 weeks following randomization

### Patient 1

70+ years-old, female, history of acute coronary syndrome, coronary bypass surgery, ischemic heart failure since 2013, EF 46%, permanent atrial fibrillation. One week prior to screening was treated by Losartan 50 mg daily, beta blockers and mineralocorticoid receptor antagonists not taken, Furosemide 20 mg daily. At screening: potassium 3.3 mmol/L, NTproBNP 8779 pg/ml; medication - Losartan 50 mg daily, Spironolactone 12.5 mg daily, Furosemide 40 mg daily; intravenous loop diuretics administered from 10 to 13 Jan 2020. Pre-Randomization: Blood pressure 124/70 mmHg, heart rate 60 beats/min, NTproBNP 4650 pg/ml, Losartan 50 mg daily, Spironolactone 12.5 mg daily, Torsemide 10 mg daily. Post-Randomization: Losartan 75 mg daily, Bisoprolol started 5 mg daily, Spironolactone 12.5 mg daily, Torsemide 10 mg daily. Visit 3 (1 week after randomization): Potassium 3.9 mmol/Litter, NTproBNP increased 15655 pg/ml, hemoglobin down to 92. Losartan 75 mg daily, Bisoprolol down to 2,5 mg daily, Spironolactone 12.5 mg daily, Torsemide up 15 mg daily. Visit 4 (3 weeks after randomization): NTproBNP still high 15033, Blood pressure 137/71 mmHg, heart rate 68 beats/min. Medications - Losartan up to 150 mg, Beta blockers stopped, Spiro up to 25 mg daily, Torsemide 20 mg daily. V5: NTproBNP 6901 pg/ml, blood pressure 125/70 mmHg, heart rate 71 beats /min. Medications - Losartan 150 mg daily, beta blockers stopped, Spironolactone 25 mg daily, Torsemide 20 mg daily. Patient feels better.

In this case a patient's NTproBNP increase triggered a decrease in beta blockers and increase in diuretics averting a worsening heart failure event

### Patient 2

50+ years-old female, history of acute coronary syndrome without revascularization, ischemic heart failure de novo, EF=33%, Diabetes mellitus, Angina Pectoris 2+. 1 week prior to screening - Irbesartan 150 mg daily, beta blockers and mineralocorticoid receptor antagonists were not taken. Screening: potassium 4.5 mmol/l, NTproBNP 2723 pg/ml, medication - Enalapril 10mg daily, Spironolactone 12,5mg daily, Furosemide 80 mg daily, intravenous loop diuretics administered from 28 to 31 Aug 2020, intravenous nitroglycerine administered from 28 to 29 Aug 2020. Pre-Randomization: Blood pressure 140/85 mmHg, heart rate 84 beats/min, NTproBNP 1798pg/ml, potassium 4.5mmol/l, Enalapril 20 mg daily, Spironolactone 12.5 mg daily, Torasemide 10 mg daily. Post-Randomization: Enalapril 20 mg daily, Bisoprolol started 5 mg daily, Spironolactone 12.5 mg daily, Torasemide 10 mg daily. Visit 3 (1 week after randomization): Potassium raised to 5.9 mmol/Litter, NTproBNP increased 2321pg/ml. Enalapril 20mg daily, Bisoprolol stayed 5 mg daily, Spironolactone stopped, Torasemide up to 30 mg daily. Visit 4 (2 weeks after randomization): Potassium 5.4 mmol/l, NTproBNP increased 3484, Heart failure status worsened - Rales 1/3, Jugular veins pressure 6-10, Blood pressure 140/90 mmHg, heart rate 90 beats/min. Medications - Enalapril 20 mg daily, Beta blockers stopped, Spironolactone stopped, Torasemide up to 40mg daily. V5 (3 weeks after randomization): NTproBNP 2687 pg/ml, potassium 5.3 mmol/l, blood pressure 138/86 mmHg, heart rate 90 beats/min. Medications - Enalapril 20mg daily, beta blockers stopped, Spironolactone stopped, Torasemide 40 mg daily. Patient feels better. On visit 6 HF status improved.

In this case a patient's NTproBNP increase triggered a decrease in beta blockers and increase in diuretics averting a worsening heart failure event

### Patient 3

60+ years-old female, non-ischemic heart failure since 2014, EF=32%, paroxysmal atrial fibrillation, 1 week prior to screening - Enalapril 10 mg, beta blockers and mineralocorticoid receptor antagonists were not taken, Torasemide 2.5mg daily. Screening: potassium 4.7 mmol/l, NTproBNP 3068 pg/ml, medication - Enalapril 20 mg daily, Spironolactone 12,5mg daily, Furosemide 80mg daily, intravenous loop diuretics administered from 13 to 17 Jan 2021. Pre-Randomization: Blood pressure 110/60 mmHg, heart rate 90 beats/min, NTproBNP 1595 pg/ml, potassium 4.2 mmol/l, Enalapril 20 mg daily, Spironolactone 12.5 mg daily, Torasemide 5 mg daily. Post-Randomization: Enalapril 20 mg daily, Bisoprolol started 5 mg daily, Spironolactone 12.5 mg daily, Torasemide 10 mg daily. Visit 3 (1 week after randomization): Potassium 4.3 mmol/Litter, NTproBNP increased 1783 pg/ml. Enalapril 20 mg daily, Bisoprolol 5 mg daily, Spironolactone 12.5mg, Torasemide 5 mg daily. Visit 4 (2 weeks after randomization): Potassium 4.0 mmol/l, NTproBNP dropped to 989 pg/ml, Enalapril raised to 40mg daily, Bisoprolol increased to 10 mg daily, Spironolactone up to 25mg daily, Torasemide 5mg daily. Visit 5 (3 weeks after randomization): NTproBNP increased 3745 pg/ml, potassium increased 5.0 mmol/l, blood pressure dropped 90/55 mmHg, heart rate 54 beats/min, heart failure status worsened, renal deterioration. Medications - Enalapril down 20 mg daily, Bisoprolol down 2.5mg daily, Spironolactone 25mg daily, Torasemide increased 15 mg daily. Visit 6 (6 weeks after randomization): NTproBNP - 419 pg/ml, potassium 4.1, blood pressure 100/65 mmHg, heart rate 55 beats/min. Medications - Enalapril 20 mg daily, Bisoprolol 2.5mg daily, Spironolactone 25 mg daily, Torasemide 15 mg daily. Patient felt better. In this case a patient's NTproBNP increase together with decrease in pulse and blood pressure triggered a decrease in beta blockers and increase in diuretics averting a worsening heart failure event

### Patient 4

40+ years-old male, history of stroke, diabetes mellitus on insulin and diet, acute coronary syndrome with percutaneous coronary intervention, ischemic heart failure since March 2021, EF=32%. 1 week prior to screening - Enalapril 5 mg, beta blockers and mineralocorticoid receptor antagonists were not taken, Torasemide 2.5mg daily. Screening: potassium 4.8 mmol/l, NTproBNP 8962 pg/ml, medication - Losartan 50 mg daily, Spironolactone 25 mg daily, Furosemide 80 mg daily, intravenous loop diuretics administered from 22 to 27 Jun 2021. Pre-Randomization: Blood pressure 130/85 mmHg, heart rate 80 beats/min, NTproBNP 5699pg/ml, potassium 4.07 mmol/l, Losartan 50 mg daily, Spironolactone 25 mg daily, Torasemide 10 mg daily. Post-Randomization: Losartan 75mg daily, Bisoprolol started 5 mg daily, Spironolactone 25 mg daily, Torasemide 10 mg daily. Visit 3 (1 week after randomization): Potassium 4.4 mmol/Litter, NTproBNP increased 5761 pg/ml. Lozartan 75mg daily, Bisoprolol 5 mg daily, Spironolactone 25mg, Torasemide up to 12,5mg daily. Visit 4 (2 weeks after randomization): Potassium 4.7 mmol/l, NTproBNP increased 7521pg/ml, Blood pressure 125/70 mmHg, heart rate 85 beats/min, heart failure status worsened. Medications: Losartan up to 150 mg daily, Bisoprolol down 2.5mg daily, Spironolactone up to 50mg daily, Torasemide up to 20 mg daily. Visit 5 (3 weeks after randomization): potassium increased 5.3mmol/l , NTproBNP 4303pg/ml. Blood pressure 120/70 mmHg, heart rate 85 beats/min, Medications - Losartan 150 mg daily, Bisoprolol 2.5 mg daily, Spironolactone 50 mg daily, Torasemide 20 mg daily. Patient felt better.

In this case a patient's NTproBNP increase triggered a decrease in beta blockers and increase in diuretics averting a worsening heart failure event

### Patient 5

50+ years-old male, history of diabetes mellitus on insulin and diet, obesity, persistent atrial fibrillation, non-ischemic heart failure since 2019, EF=23%, 1 week prior to screening - Lisinopril 10 mg, Spironolactone 25mg daily, Furosemide 80mg daily. Screening: potassium 3.9 mmol/l, NTproBNP 10337 pg/ml, Entresto 100 mg daily, Spironolactone 25 mg daily, Furosemide 80 mg daily, intravenous loop diuretics administered from 17 to 25 Jan 2022. Pre-Randomization: Blood pressure 119/72 mmHg, heart rate 101 beats/min, NTproBNP 4850 pg/ml, potassium 3.61 mmol/l, Entresto 100 mg daily, Spironolactone 25 mg daily, Furosemide 80 mg daily. Post-Randomization: Entresto 200mg daily, Bisoprolol started 5 mg daily, Eplerenone 25 mg daily, Furosemide 80 mg daily. Visit 3 (1 week after randomization): Potassium 4.58 mmol/Litter, NTproBNP 4188 pg/ml. Entresto 200mg daily, Bisoprolol 5 mg daily, Eplerenone 25 mg daily, Torasemide 20 mg daily. Visit 4 (2 weeks after randomization): Potassium increased 5.06 mmol/l, NTproBNP increased 7417 pg/ml, Blood pressure 118/68 mmHg, heart rate 118 irregular beats/min, heart failure status worsened. Medications: Entresto 200mg daily, Bisoprolol down to 2.5 mg daily, Eplerenone 25 mg daily, Torasemide up to 30 mg daily. Visit 5 (3 weeks after randomization): potassium increased 5.39 mmol/l , NTproBNP down to 5203 pg/ml. Blood pressure 110/68 mmHg, heart rate 108 beats/min, Medications - Entresto down to 100 mg daily, Bisoprolol 2.5 mg daily, Eplerenone stopped, Torasemide up to 40 mg daily. Heart Failure status improved. Patient felt better.

In this case a patient's NTproBNP increase triggered a decrease in beta blockers and increase in diuretics averting a worsening heart failure event

### Patient 6

60+ years-old, female, stroke, Acute coronary syndrome, not revascularized, ischemic HF since 2019, LVEF 50%. One week prior to screening Enalapril 5mg, Spironolactone 25mg, Torasemide 2,5mg. At screening: Blood pressure 150/95 mmHg, heart rate 87 beats/min, potassium 4.93 mmol/L, NTproBNP 5031 pg/ml. Medication: Candesartan 16 mg daily, Spironolactone 25 mg daily, furosemide 40 mg. Pre-Randomization: Blood pressure 135/85 mmHg, heart rate 76 beats/min, potassium 4.5 mmol/L, NTproBNP 2097 pg/ml. Candesartan 16 mg daily, Spironolactone 25 mg daily, Torasemide 10 mg, intravenous loop diuretics administered from 11 Jun to 15 Jun 2021.

Post-Randomization: Candesartan 16 mg daily, Bisoprolol started 5 mg, Spironolactone 25 mg daily, Torasemide 10 mg. At Visit 3 (1 week after randomization): Blood pressure 120/75 mmHg, heart rate 64 beats/min, Potassium 4.4 mmol/L, NTproBNP up 3999 pg/ml. Candesartan 16 mg daily, Bisoprolol 5mg, Spironolactone 25 mg daily, Torasemide 10mg. Visit 4 (2 weeks after randomization):_Blood Pressure 115/65 mmHg, heart rate 80 beats/min. Potassium up 5.93 mmol/L, NTproBNP up 6428 pg/ml, eGFR down to 35ml/min/1,73m2. Candesartan 16 mg daily, bisoprolol down 2,5mg, Spironolactone stopped, Torasemide up 20 mg. Visit 5 (3 weeks after randomization):_Blood Pressure 118/80 mmHg, heart rate 54 beats/min. Potassium 5.1 mmol/L, NTproBNP 2392 pg/ml. Candesartan 16 mg daily, bisoprolol 2,5 mg, Torasemide 20mg. Patient feels better.

In this case a patient's NTproBNP increase triggered a decrease in beta blockers and increase in diuretics averting a worsening heart failure event

### Patient 7

80+ years-old female, history of ischemic heart failure since 2019, EF=36%, 1 week prior to screening - no medications. Screening: potassium 3.38 mmol/l, NTproBNP 7102 pg/ml, Enalapril 10 mg daily, Spironolactone 12,5 mg daily, Furosemide 40 mg daily, intravenous loop diuretics administered from 20 to 24 November 2019. Pre-Randomization: Blood pressure 130/70 mmHg, heart rate 78 beats/min, NTproBNP 6195 pg/ml, potassium 3.93 mmol/l, Enalapril 10 mg daily, Spironolactone 12,5 mg daily, Furosemide 40 mg daily. Post-Randomization Enalapril 20 mg daily, Spironolactone 12,5 mg daily, Metoprolol started 100 mg daily, no loop diuretics. Visit 3 (1 week after randomization): Potassium 4.78 mmol/Litter, NTproBNP increased 9000 pg/ml. Enalapril 20 mg daily, Spironolactone 12,5 mg daily, Metoprolol 100 mg daily, Furosemide 20 mg. Visit 4 (2 weeks after randomization): Potassium 4.26 mmol/l, NTproBNP >9000 pg/ml, Blood pressure 110/60 mmHg, heart rate 82 beats/min. Medications: Enalapril up to 40 mg daily, Spironolactone 25 mg daily, Metoprolol down 50 mg daily, Furosemide up to 40mg. Visit 5: potassium 4.23 mmol/l , NTproBNP down to 6543 pg/ml. Blood pressure 110/70 mmHg, heart rate 78 beats/min. Medications: Enalapril 40 mg daily, Spironolactone 25 mg daily, Metoprolol 50 mg daily Furosemide 40mg daily. Patient felt better.

In this case a patient's NTproBNP increase triggered a decrease in beta blockers and increase in diuretics averting a worsening heart failure event

### Patient 8

70+ years-old, female, with non-ischemic de novo heart failure, permanent Atrial Fibrillation, EF=48%. One week prior to screening Metoprolol 25 mg daily, Torasemide 5 mg daily. At screening: Blood pressure 126/84 mmHg, heart rate 118 beats/min, potassium 3.7 mmol/L, NTproBNP 4860 pg/ml. Medication: carvedilol 12,5 mg daily, Spironolactone 12.5 mg daily, Torasemide 10 mg daily; intravenous loop diuretics administered from 13 to 15 May 2020. Pre-Randomization: Blood pressure 132/84 mmHg, heart rate 84 beats/min, potassium 4.4 mmol/L, NTproBNP 2900 pg/ml. Carvedilol 25 mg daily, Spironolactone 12.5 mg daily, Torsemide 10 mg daily. Post-Randomization: Valsartan 160 mg daily, Carvedilol 25 mg daily, Spironolactone 12.5 mg daily, Torsemide 10 mg daily. At Visit 3 (1 week after randomization): Potassium 4.6 mmol/L, NTproBNP 1986 pg/ml. Valsartan 160 mg daily, Carvedilol 25 mg daily, Spironolactone 12.5 mg daily, Torsemide 10 mg daily. Visit 4 (2 weeks after randomization): Blood Pressure 134/80 mmHg, heart rate 96 beats/min. Potassium 4.3 mmol/L, NTproBNP 1876 pg/ml, Valsartan 160 mg daily, Carvedilol up-titrated 50 mg daily, Spironolactone up 25mg daily, Torsemide 5 mg daily. Visit 5 (3 weeks after randomization): Blood pressure 120/72 mmHg, heart rate 88 beats/min. Weight +2,2kg, Heart Failure status worsened. Potassium 4.8 mmol/L, NTproBNP increased 3662 pg/ml. Valsartan 160 mg daily, Carvedilol decreased 37,5 mg daily, Spironolactone 25 mg daily, Torsemide up 10 mg daily. Visit 6 (6 weeks after randomization): Blood Pressure 102/76 mmHg, heart rate 72/min. Potassium 5.1 mmol/L, NTproBNP 781 pg/ml. Valsartan 160 daily, Carvedilol 37,5 mg daily, Spironolactone 25mg daily, Torsemide 5 mg. Heart Failure status improved. Patient feels better.

In this case a patient's NTproBNP increase triggered a decrease in beta blockers and increase in diuretics averting a worsening heart failure event

### Patient 9

30+ years-old, female, non-ischemic Heart Failure since Feb 2018, EF=38%. One week prior to screening Lisinopril 2,5 mg daily, spironolactone 25 mg daily, furosemide 80mg. At screening: Blood pressure 120/80 mmHg, heart rate 122 beats/min, potassium 3.9 mmol/L, NTproBNP 2712 pg/ml. Medication: Lisinopril 2,5 mg daily, Spironolactone 12.5 mg daily; intravenous loop diuretics administered from 19 Dec to 01 Jan 2020. Pre-Randomization: Blood pressure 130/80 mmHg, heart rate 68 beats/min, potassium 4.1 mmol/L, NTproBNP 1645 pg/ml. Lisinopril 2,5 mg daily, Spironolactone 12.5 mg daily, Furosemide 160 mg. Post-Randomization: Lisinopril 17,5 mg daily, Spironolactone 12.5 mg daily, Carvedilol started 25 mg daily, Furosemide 160 mg daily. At Visit 3 (1 week after randomization): Blood pressure 120/80 mmHg, heart rate 90 beats/min, Potassium 4.2 mmol/L, NTproBNP 1443 pg/ml, Lisinopril 17,5 mg daily, Spironolactone 25 mg daily, Carvedilol 25 mg daily, Furosemide 80 mg daily. Visit 4 (2 weeks after randomization): Blood Pressure 110/70 mmHg, heart rate 82 beats/min. Potassium 4.4 mmol/L, NTproBNP 1340 pg/ml. Lisinopril 35 mg daily, Spironolactone 25 mg daily, Carvedilol up 50 mg daily, Furosemide 80 mg daily.

Visit 5 (3 weeks after randomization): Blood pressure 90/70 mmHg, heart rate 98 beats/min, Heart Failure status worsened. NYHA III, edema +1. Potassium 5.1 mmol/L, NTproBNP up 2640 pg/ml. Lisinopril 10 mg daily, Spironolactone 12,5 mg daily, Carvedilol down to 25 mg daily, Furosemide up 160 mg daily. Visit 6 (6 weeks after randomization): Blood Pressure 90/70 mmHg, heart rate 74/min. Potassium 4.9 mmol/L, NTproBNP 1757 pg/ml. Lisinopril 10 mg daily, Spironolactone 12,5 mg daily, Carvedilol down 12,5 mg daily, Furosemide 40 mg daily. Visit 7: Blood Pressure 95/70 mmHg, heart rate 76 beats/min. Potassium 4.2 mmol/L, NTproBNP 974 pg/ml, Lisinopril 10 mg daily, Spironolactone 12,5 mg daily, Carvedilol 12,5 mg daily, Furosemide 40 mg daily. Patient feels better.

In this case a patient's NTproBNP increase triggered a decrease in beta blockers and increase in diuretics averting a worsening heart failure event

### Patient 10

60+ years-old, male, with non-ischemic HF since July 2020, LVEF 35%. One week prior to screening Losartan 50 mg, Spironolactone 25 mg daily, Furosemide 120 mg daily. At screening: Blood pressure 140/80 mmHg, heart rate 124 beats/min, potassium 4.1 mmol/L, NTproBNP 2763 pg/ml. Medication: Losartan 50 mg, Spironolactone 12.5mg daily; intravenous loop diuretics administered from 29 Aug 2020 to 07 Sep 2020. Pre-Randomization: Blood pressure 140/90 mmHg, heart rate 84 beats/min, potassium 3.9 mmol/L, NTproBNP 1696 pg/ml. Losartan 50 mg daily, Spironolactone 12.5 mg daily, Furosemide 80 mg daily. Post-Randomization: Losartan 75 mg daily, Carvedilol started 25 mg daily, Spironolactone 12.5 mg daily, Furosemide 80 mg daily. At Visit 3 (1 week after randomization): Blood pressure 130/80 mmHg, heart rate 86 beats/min. Potassium 4.1 mmol/L, NTproBNP 1078 pg/ml. Losartan 75 mg daily, Carvedilol 25 mg daily, Spironolactone 12.5 mg daily, Furosemide 40 mg daily. Visit 4 (2 weeks after randomization):_Blood Pressure 140/90 mmHg, heart rate 96 beats/min. Potassium 4.0 mmol/L, NTproBNP 1041 pg/ml. Losartan 150 mg daily, Carvedilol up 50 mg daily, Spironolactone up 25 mg daily, Furosemide 40 mg daily.

Visit 5 (3 weeks after randomization): Blood pressure 130/90 mmHg, heart rate 82 beats/min. Potassium 4.0 mmol/L, NTproBNP increased 1826 pg/ml. Losartan 75 mg, Carvedilol decreased 25 mg, Spironolactone 12,5 mg, Furosemide 80 mg daily. Visit 6 (6 weeks after randomization): Blood Pressure 130/80 mmHg, heart rate 76 beats/min. Potassium 4.8 mmol/L, NTproBNP 996 pg/ml. Losartan 150, Carvedilol 50 mg, Spironolactone 25 mg, Furosemide 40 mg. Patient feels better.

In this case a patient's NTproBNP increase triggered a decrease in beta blockers and increase in diuretics averting a worsening heart failure event

### Patient 11

60+ years-old, female, with non-ischemic HF since May 2019, LVEF 38%. One week prior to screening Losartan 50 mg, Spironolactone 25mg daily, Torasemide 50 mg daily. At screening: Blood pressure 150/100 mmHg, heart rate 122 beats/min, potassium 4.2 mmol/L, NTproBNP >9000 pg/ml. Medication: Losartan 50 mg, Spironolactone 12.5mg daily; intravenous loop diuretics administered from 31 Aug 2020 to 07 Sep 2020. Pre-Randomization: Blood pressure 150/90 mmHg, heart rate 92 beats/min, potassium 4.0 mmol/L, NTproBNP 2321 pg/ml. Losartan 50 mg daily, Spironolactone 12.5 mg daily, Torasemide 50 mg daily. Post-Randomization: Losartan 75 mg daily, Carvedilol started 25 mg daily, Spironolactone 12.5 mg daily, Torasemide 50 mg daily. At Visit 3 (1 week after randomization): Blood pressure 150/90 mmHg, heart rate 87 beats/min. Potassium 4.4 mmol/L, NTproBNP 1633 pg/ml. Losartan 75 mg daily, Carvedilol 25 mg daily, Spironolactone 12.5 mg daily, Furosemide 40 mg daily. Visit 4 (2 weeks after randomization): Blood Pressure 160/90 mmHg, heart rate 94 beats/min. Potassium 4.2 mmol/L, NTproBNP 1049 pg/ml. Losartan up 150 mg, Carvedilol up 50mg daily, Spironolactone up 25mg daily, Furosemide 40 mg daily. Visit 5 (3 weeks after randomization):_Blood pressure 150/90 mmHg, heart rate 100 beats/min, Heart Failure status worsened: NYHA III, edema +1. Potassium 4.5 mmol/L, NTproBNP increased 2108 pg/ml, eGFR dropped from 60,8 to 47,2. Losartan 75 mg, Carvedilol down 25 mg, Spironolactone 12,5mg, Furosemide 120 mg daily. Visit 6 (6 weeks after randomization): Blood Pressure 130/100 mmHg, heart rate 91/min. Potassium 4.4 mmol/L, NTproBNP 1085 pg/ml. Losartan 150, Carvedilol 50 mg, Spironolactone 25mg, Furosemide 80 mg. Patient feels better. Visit 6 (6 weeks after randomization): Blood Pressure 140/110 mmHg, heart rate 92/min. Potassium 4.5 mmol/L, NTproBNP 756 pg/ml. Losartan 150, Carvedilol 50 mg, Spironolactone 25mg, Furosemide 80 mg. patient feels better.

In this case a patient's NTproBNP increase triggered a decrease in beta blockers and increase in diuretics averting a worsening heart failure event

### Patient 12

60+ years-old, male, non-ischemic HF de-novo, EF=24%. One week prior to screening no medication. At screening: Blood pressure 144/77 mmHg, heart rate 50 beats/min, potassium 4.98 mmol/L, NTproBNP 9000 pg/ml. Medication: Enalapril 20 mg daily, Spironolactone 12.5 mg daily, Furosemide 80 mg. Pre-Randomization: Blood pressure 151/81 mmHg, heart rate 91 beats/min, potassium 4.3 mmol/L, NTproBNP 5656 pg/ml. Enalapril 20 mg daily, Spironolactone 12.5 mg daily, Furosemide 80 mg. Post-Randomization: Enalapril 20 mg daily, Spironolactone 25 mg daily, Bisoprolol started 5 mg daily, Furosemide 80 mg daily. At Visit 3 (1 week after randomization): Blood pressure 150/114 mmHg, heart rate 93 beats/min, Potassium 4.27 mmol/L, NTproBNP 7122 pg/ml. Enalapril 20 mg daily, Spironolactone 25 mg daily, Bisoprolol 5 mg daily, Furosemide 80 mg daily. Visit 4 (2 weeks after randomization): Blood Pressure 133/97 mmHg, heart rate 85 beats/min. Potassium 4.19 mmol/L, NTproBNP 4854 pg/ml. Enalapril 40 mg daily, Spironolactone 25 mg daily, Bisoprolol 10 mg daily, Furosemide 80 mg daily. Visit 5 (3 weeks after randomization): Blood Pressure 138/95 mmHg, heart rate 60 beats/min. Potassium 3.69 mmol/L, NTproBNP up 6800 pg/ml. Enalapril 40 mg daily, Spironolactone 25 mg daily, Bisoprolol 10 mg daily, Furosemide 80 mg daily.

Visit 6 (6 weeks after randomization): Blood Pressure 161/116 mmHg, heart rate 61/min. HF status worsened to NYHA III. Potassium 3.35 mmol/L, NTproBNP >9000 pg/ml, Enalapril 40 mg daily, Spironolactone 25 mg daily, Bisoprolol down 5 mg daily, Furosemide up 200 mg daily. Visit 7: Blood Pressure 163/109 mmHg, heart rate 70 beats/min. Potassium 3.4 mmol/L, NTproBNP 7068 pg/ml. Enalapril 40 mg daily, Spironolactone 25 mg daily, Bisoprolol 5 mg daily, Furosemide 200 mg daily. Patient feels better.

In this case a patient's NTproBNP increase triggered a decrease in beta blockers and increase in diuretics averting a worsening heart failure event

### Patient 13

50+ years-old, male, ACS without revascularisation, HF since May 2021 when 1 hospitalization for HF occurred; LVEF33%, permanent AF
1 week prior: Losartan 50 mg, Torasemide 5 mg
SCR: K 4.15; NTproBNP 3154; Losartan 75 mg, Eplerenone 25 mg; Furosemide 40 mg
Pre-R: Troponin I negative; K 4.64; NTproBNP 1320 -waiver given. Meds - Losartan 75 mg, Eplerenone 25 mg, Torasemide 10 mg
Post-R: 4 days IV diuretics; Losartan 75 mg, Biso 5 mg, Eplerenone 25 mg, Torasemide 10 mg, Digoxin
V3: no labs,_BP 125/90 mmHg, 80 bpm, Losartan 75 mg, Biso 5 mg, Spiro 25 mg, Torsemide 10 mg
V4: K 4.69, NTproBNP up 9605, WBC up 11,6. BP 145/95, HR 98. Weight up by 2,6 kg, HF status worsened. Losartan 150 mg, Biso down to 2,5 mg, Eplerenone 50 mg, Torasemide up 20 mg. WHF event to be reported!
V5: K 4.08, NTproBNP 2702, eGFR dropped from 55 to 36. BP 128/80 mmHg, 82 bpm; worsening renal function to be reported! Meds - Losartan 150 mg, Biso 2,5 mg, Eplerenone 50 mg, Torasemide 20 mg.
V6: K 4.83, NTproBNP 674, BP 130/80 mmHg, HR 74 bpm, Losartan 150 mg, Biso 2.5 mg, Eplerenone 50 mg, Torsemide 20 mg
V7: K 4.89, NTproBNP 891, 122/76 mmHg, 76 bpm, Losartan 150 mg, Biso 2.5 mg, Eplerenone 50 mg, Torsemide 20 mg

Patient was managed with NTproBNP measurements, medications were down titrated and diuretics increased based on NTproBNP increase and the patient stabilized without a readmission.

### Patient 14

50+years-old, male, ACS without revascularization, ischemic HF since 2019, LVEF 30%, 1 hospitalization in March 2021, paroxysmal AFib
1 week prior: Losartan 50 mg, diuretics 5 mg, Scr: K 4.86, NTproBNP 10333, Lisinopril 2.5 mg, Spiro 25 mg, Furosemide 80 mg
Pre-R: Troponin I negative, K 3.75, NTproBNP 3926, BP 120/70, HR 80. Lisinopril 10 mg, Spiro 25 mg, Furosemide 40 mg
Post-R: 7 days IV loop, Lisinopril 16.5 mg, Bisoprolol 5 mg, Spiro 25 mg, Furosemide 40 mg.
V3: no data for lab, HF assessment; only meds: Lisinopril 16.5 mg, Bisoprolol 5 mg, Spiro 25 mg, Furosemide 40 mg
V4: K 4.97, NTproBNP up 6558, 103/68 mmHg, 84 bpm, Lisinopril 16.5 mg, Bisoprolol down 2.5 mg, Spiro up 50 mg, Furosemide up 80 mg; WHF rales 1/3, weight up by 1,8 kg, NTproBNP increased. AE reported.
V5: K 5.06, NTproBNP 4939, 100/65 mmHg, 79 bpm, Lisinopril 16.5 mg, Bisoprolol 2.5 mg, Spiro 50 mg, Furosemide up 100 mg
V6: K 4.29, NTproBNP 2409, 102/60 mmHg, 74 bpm, Lisinopril 16.5 mg, Bisoprolol 2.5 mg, Spiro 50 mg, Furosemide 100 mg
AE: WHF - start 05 Dec 2021
AE2: bruised ankle 12 Dec 2021
V7: K 4.71, NTproBNP 2302, BP 100/62, HR 66. Meds - Lisinopril 16.5 mg, Bisoprolol 2.5 mg, Spiro 50 mg, Furosemide 100 mg

### Patient 15

50+ years-old, male, DM on insulin and diet, non-ischemic HF since 2019, LVEF 23%, 1 hospitalization Feb 2021, persistent AF, obesity
1 week prior: Lisinopril 10 mg, Spironolactone 25 mg, Furosemide 80 mg Scr: K 3.9, NTproBNP 10337
Pre-R: Troponin I negative, K 3.61, NTproBNP 4850, BP 119/72, HR 101. Entresto 100 mg, Spiro 25 mg, Furosemide 80 mg
Post-R: 9 days IV diuretics, Entresto 200 mg, Bisoprolol 5mg, Eplerenone 25 mg, Furosemide 80 mg
V3: K 4.58, NTproBNP 4188, 115/70 mmHg, 99 bpm, Entresto 200 mg, Bisoprolol 5 mg, Eplerenone 25 mg, Torsemide 20 mg
V4: K 5.06, NTproBNP up 7417, 118/68 mmHg, 118 bpm (AF). Entresto 200 mg, Bisoprolol down 2.5 mg, Eplerenone 25 mg, Torasemide up 30 mg
V5: K 5.39, NTproBNP 5203, 110/68 mmHg, 108 bpm, Entresto 100 mg, Biso 2.5 mg, MRA stopped, Torasemide 40 mg
V6: K 4.9, NTproBNP 2823, 110/68 mmHg, 89 bpm, Entresto 100 mg, Biso 2.5 mg, Torsemide 40 mg;
V7: K 5.3, NTproBNP 2805, BP 112/66 mmHg, 78 bpm, Entresto 100 mg, Biso 2.5 mg, Torsemide 40 mg
V8: Entresto 100 mg, Biso 2.5 mg, Torsemide 40 mg Spironolactone 25 mg
AE: WHF start date 13.Feb 2022
AE hyperK start date 20 Feb2022, entresto dose reduced, eplerenone stopped

### Patient 16 (Control)

6X years-old, male, history of Diabetes Mellitus Type II, non-ischemic heart failure since 2019, EF 27%. One week prior to screening was treated by Entresto 50 mg daily, beta blockers and mineralocorticoid receptor antagonists not taken, no loop diuretics. At screening: potassium 4.18 mmol/L, NTproBNP 2788 pg/ml; medication - Entresto 100 mg daily, Spironolactone 25 mg daily, Furosemide 120 mg daily; intravenous loop diuretics administered from 11 to 13 Jan 2022. Pre-Randomization: Blood pressure 135/68 mmHg, heart rate 74 beats/min, NTproBNP 1687 pg/ml, Entresto 200 mg daily, Spironolactone 25 mg daily, Furosemide 80 mg daily. Post-Randomization: Entresto 200 mg daily, Bisoprolol started 5 mg daily, Spironolactone 25 mg daily, Furosemide 40 mg daily. Visit 3 (1 week after randomization): Potassium 4,52 mmol/Litter, NTproBNP 1214 pg/ml, Entresto 200 mg daily, Bisoprolol 5 mg daily, Spironolactone 25 mg daily, Furosemide 40 mg daily. Visit 4 (3 weeks after randomization): NTproBNP 1211, Blood pressure 139/88 mmHg, heart rate 69 beats/min. Medications - Entresto up to 400 mg, Bisoprolol up to 10 mg daily, Spiro 25 mg daily, Furosemide 40 mg daily. V5: NTproBNP up 1838 pg/ml, blood pressure 123/69 mmHg, heart rate 62 beats /min. NYHA class worsened from I to II. Medications - no change: Entresto 400 mg daily, Bisoprolol 10 mg, Spironolactone 25 mg daily, Furosemide 40 mg daily. Visit 6 (6 weeks after randomization): Heart Failure status worsened: NYHA class II, edema worsened to +1, rales 1/3, Jugular veins pressure 6-10, weight increased by 3,7 kg. Blood pressure 153/62 mmHg, heart rate 68 beats/min. Potassium 5,12 mmol/Litter, NTproBNP up 2933 pg/ml. Medications -Entresto 400 mg daily, Bisoprolol 10 mg daily, Spironolactone 25 mg daily, Furosemide increased to 80 mg. Visit 7 (90 days after randomization): **Heart Failure status was getting worse** - NYHA class III, edema +2, Jugular veins pressure 6-10, weight increased by 0,5kg. Blood pressure up 166/78 mmHg, heart rate 68 beats/min. Potassium up 5,9 mmol/Litter, NTproBNP up 3667 pg/ml. Medications -Entresto 400 mg daily, Bisoprolol 10 mg daily, Spironolactone 25 mg daily, Furosemide increased to 160 mg.

### Patient 17 (Control)

5X years-old male, history of ischemic heart failure since April 2020, EF=40%, Diabetes mellitus, obesity, permanent Atrial Fibrillation. 1 week prior to screening - Bisoprolol 5 mg daily, Spironolactone 12,5 mg daily. Screening: potassium 4.96 mmol/l, NTproBNP 6024 pg/ml, medication-Bisoprolol 5 mg daily, Spironolactone 12,5mg daily, Torasemide 40 mg daily, intravenous loop diuretics administered from 25 Feb to 03 Mar 2021. Pre-Randomization: Blood pressure 105/70 mmHg, heart rate 68 beats/min, NTproBNP 2028 pg/ml, potassium 4.9mmol/l, Bisoprolol 5 mg, Spironolactone 12.5 mg daily, Torasemide 40 mg daily. Post-Randomization: Losartan 75 mg daily, Bisoprolol 5 mg daily, Spironolactone 12.5 mg daily, Torasemide 40 mg daily. Visit 3 (1 week after randomization): Potassium 5.1 mmol/l, NTproBNP increased 3837 pg/ml. Losartan 75 mg daily, Bisoprolol stayed 5 mg daily, Spironolactone 12,5 mg, Torasemide 40 mg daily. Visit 4 (2 weeks after randomization): Potassium 5.2 mmol/l, NTproBNP 3793 (increase more than 10% of post-randomization level), Blood pressure 115/70 mmHg, heart rate 76 beats/min. Medications - Losartan 75 mg daily, Bisoprolol up 10 mg, Spironolactone up 25 mg, Torasemide 40 mg daily. V5 (3 weeks after randomization): NTproBNP up 8734 pg/ml, potassium 4.0 mmol/l, blood pressure 105/65 mmHg, heart rate 72 beats/min. Medications - no change: Losartan 75 mg daily, Bisoprolol 10 mg, Spironolactone 25 mg, Torasemide 40 mg daily. **On visit 6 (6 weeks after randomization) patient felt worse:** NYHA class III, edema +2, weight increased by 1,5 kg, Potassium 4.6 mmol/l, NTproBNP up 9405 pg/ml, Blood pressure 112/69 mmHg, heart rate 78 beats/min. Medications - Losartan 75 mg daily, Bisoprolol 10 mg, Spironolactone 25 mg, Torasemide 40 mg daily.

### Patient 18 (Control)

7X years-old female, history of acute coronary syndrome without revascularization, ischemic heart failure since 2017, EF=43%, paroxysmal atrial fibrillation, diabetes mellitus type 2. 1 week prior to screening - Losartan 50 mg, beta blockers and mineralocorticoid receptor antagonists were not taken. Screening: potassium 4.6 mmol/l, NTproBNP 22208 pg/ml, medication - Losartan 75 mg daily, Spironolactone 25mg daily, Furosemide 120 mg daily, intravenous loop diuretics administered from 17 to 23 May 2021. Pre-Randomization: Blood pressure 120/70 mmHg, heart rate 76 beats/min, NTproBNP 8850 pg/ml, potassium 4.2 mmol/l, Losartan 50 mg daily, Spironolactone 25 mg daily, Torasemide 10 mg daily. Post-Randomization: Losartan 75 mg daily, Bisoprolol started 5 mg daily, Spironolactone 25 mg daily, Torasemide 10 mg daily. Visit 3 (1 week after randomization): Potassium 4.8 mmol/Litter, NTproBNP 5632 pg/ml. Losartan 75 mg daily, Bisoprolol 5 mg daily, Spironolactone 25 mg, Torasemide 10 mg daily. Visit 4 (2 weeks after randomization): Potassium 4.68 mmol/l, NTproBNP up 6433 pg/ml, blood pressure 135/85 mmHg, heart rate 64 beats/min. Losartan up to 150 mg daily, Bisoprolol increased to 10 mg daily, Spironolactone up to 50 mg daily, Torasemide 10 mg daily. Visit 5 (3 weeks after randomization): NTproBNP increased 6768 pg/ml, potassium 4.68 mmol/l, blood pressure 120/70 mmHg, heart rate 63 beats/min, weight increased by 1,1 kg. Medications - Losartan 150 mg daily, Bisoprolol 10 mg daily, Spironolactone 50 mg daily, Torasemide 10 mg daily. Visit 6 (6 weeks after randomization): **patient status getting worse.** NYHA IV, edema +2, rales 1/3, jugular veins pressure 6-10, NTproBNP up 15304 pg/ml, blood pressure 135/80 mmHg, heart rate 95 beats/min, weight increased by 3,1 kg. Worsening heart failure reported. Medications - Losartan 150 mg daily, Bisoprolol stopped, Spironolactone 50 mg daily, Torasemide up 30 mg daily.

### References

1. Chioncel O, Mebazaa A, Harjola VP, Coats AJ, Piepoli MF, Crespo-Leiro MG, Laroche C, Seferovic PM, Anker SD, Ferrari R, Ruschitzka F, Lopez-Fernandez S, Miani D, Filippatos G, Maggioni AP. Clinical phenotypes and outcome of patients hospitalized for acute heart failure: the ESC Heart Failure Long-Term Registry. Eur J Heart Fail. 2017;19: 1242-1254.
2. Granger BB, Kaltenbach LA, Fonarow GC, Allen LA, Lanfear DE, Albert NM, Al-Khalidi HR, Butler J, Cooper LB, Dewald T, Felker GM, Heidenreich P, Kottam A, Lewis EF, Piña IL, Yancy CW, Granger CB, Hernandez AF, Devore AD. Health System-Level Performance in Prescribing Guideline-Directed Medical Therapy for Patients With Heart Failure With Reduced Ejection Fraction: Results From the CONNECT-HF Trial. J Card Fail. 2022 Aug;28(8):1355-1361.
3. Butler J, Yang M, Sawhney B, Chakladar S, Yang L, Djatche LM. Treatment patterns and clinical outcomes among patients <65 years with a worsening heart failure event. Eur J Heart Fail. 2021 Aug;23(8): 1334-1342.
4. Greene SJ, Butler J, Albert NM, DeVore AD, Sharma PP, Duffy CI, Hill CL, McCague K, Mi X, Patterson JH, Spertus JA, Thomas L, Williams FB, Hernandez AF, Fonarow GC. Medical Therapy for Heart Failure With Reduced Ejection Fraction: The CHAMP-HF Registry. J Am Coll Cardiol. 2018 Jul 24;72(4):351-366.
5. Joseph S, Panniyammakal J, Abdullakutty J, S S, Vaikathuseril L J, Joseph J, Mattummal S, Punnose E, Unni G, Natesan S, Sivadasanpillai H. The Cardiology Society of India-Kerala Acute Heart Failure Registry: poor adherence to guideline-directed medical therapy. Eur Heart J. 2021 Dec 21:ehab793. doi: 10.1093/eurheartj/ehab793. Epub ahead of print. PMID: 34931232
6. Damasceno A, Mayosi BM, Sani M, Ogah OS, Mondo C, Ojji D, Dzudie A, Kouam CK, Suliman A, Schrueder N, Yonga G, Ba SA, Maru F, Alemayehu B, Edwards C, Davison BA, Cotter G, Sliwa K. The causes, treatment, and outcome of acute heart failure in 1006 Africans from 9 countries. Arch Intern Med. 2012 Oct 8;172(18):1386-94.
7. Čerlinskaitė K, Mebazaa A, Cinotti R, Matthay M, Wussler DN, Gayat E, Juknevičius V, Kozhuharov N, Dinort J, Michou E, Gualandro DM, Palevičiu̅tė E, Alitoit-Marrote I, Kablučko D, Bagdonaite L, Balčiu̅nas M, Vaičiulienė D, Jonauskiene I, Motieju̅naitė J, Stašaitis K, Kukulskis A, Damalakas Š, Laucevičius A, Mueller C, Kavoliu̅nienė A, Čelutkienė J; GREAT network. Readmission following both cardiac and non-cardiac acute dyspnoea is associated with a striking risk of death. ESC Heart Fail. 2021 Aug;8(4):2473-2484.
8. Logeart D, Berthelot E, Bihry N, Eschalier R, Salvat M, Garcon P, Eicher JC, Cohen A, Tartiere JM, Samadi A, Donal E, deGroote P, Mewton N, Mansencal N, Raphael P, Ghanem N, Seronde MF, Chavelas C, Rosamel Y, Beauvais F, Kevorkian JP, Diallo A, Vicaut E, Isnard R. Early and short-term intensive management after discharge for patients hospitalized with acute heart failure: a randomized study (ECAD-HF). Eur J Heart Fail. 2022 Jan;24(1):219-226.
9. Bistola V, Simitsis P, Parissis J, Ouwerkerk W, van Veldhuisen DJ, Cleland JG, Anker SD, Samani NJ, Metra M, Zannad F, Polyzogopoulou E, Keramida K, Farmakis D, Voors AA, Filippatos G. Association between up-titration of medical therapy and total hospitalizations and mortality in patients with recent worsening heart failure across the ejection fraction spectrum. Eur J Heart Fail. 2021 Jul;23(7): 1170-1181.
10. Carubelli V, Lombardi C, Specchia C, Peveri G, Oriecuia C, Tomasoni D, Di Pasquale M, Inciardi R, Garrafa E, Metra M. Adherence and optimization of angiotensin converting enzyme inhibitor/angiotensin II receptors blockers and beta-blockers in patients hospitalized for acute heart failure. ESC Heart Fail. 2021 Jun;8(3):1944-1953.
11. Carubelli V, Lombardi C, Specchia C, Peveri G, Oriecuia C, Tomasoni D, Di Pasquale M, Inciardi R, Garrafa E, Metra M. Adherence and optimization of angiotensin converting enzyme inhibitor/angiotensin II receptors blockers and beta-blockers in patients hospitalized for acute heart failure. ESC Heart Fail. 2021 Jun;8(3):1944-1953.
12. Ouwerkerk W, Teng TK, Tromp J, Tay WT, Cleland JG, van Veldhuisen DJ, Dickstein K, Ng LL, Lang CC, Anker SD, Zannad F, Hung CL, Sawhney JPS, Naik A, Shimizu W, Hagiwara N, Wander GS, Anand I, Richards AM, Voors AA, Lam CSP. Effects of combined renin-angiotensin-aldosterone system inhibitor and beta-blocker treatment on outcomes in heart failure with reduced ejection fraction: insights from BIOSTAT-CHF and ASIAN-HF registries. Eur J Heart Fail. 2020 Aug;22(8):1472-1482.
13. Felker GM, Anstrom KJ, Adams KF, Ezekowitz JA, Fiuzat M, Houston-Miller N, Januzzi JL Jr, Mark DB, Piña IL, Passmore G, Whellan DJ, Yang H, Cooper LS, Leifer ES, Desvigne-Nickens P, O'Connor CM. Effect of Natriuretic Peptide-Guided Therapy on Hospitalization or Cardiovascular Mortality in High-Risk Patients With Heart Failure and Reduced Ejection Fraction: A Randomized Clinical Trial. JAMA. 2017 Aug 22;318(8):713-720.
14. Yamaguchi T, Kitai T, Miyamoto T, Kagiyama N, Okumura T, Kida K, Oishi S, Akiyama E, Suzuki S, Yamamoto M, Yamaguchi J, Iwai T, Hijikata S, Masuda R, Miyazaki R, Hara N, Nagata Y, Nozato T, Matsue Y. Effect of Optimizing Guideline-Directed Medical Therapy Before Discharge on Mortality and Heart Failure Readmission in Patients Hospitalized With Heart Failure With Reduced Ejection Fraction. Am J Cardiol. 2018 Apr 15;121(8):969-974.
15. Gayat E, Arrigo M, Littnerova S, Sato N, Parenica J, Ishihara S, Spinar J, Müller C, Harjola VP, Lassus J, Miró Ò, Maggioni AP, AlHabib KF, Choi DJ, Park JJ, Zhang Y, Zhang J, Januzzi JL Jr, Kajimoto K, Cohen-Solal A, Mebazaa A; GREAT Network. Heart failure oral therapies at discharge are associated with better outcome in acute heart failure: a propensity-score matched study. Eur J Heart Fail. 2018 Feb;20(2):345-354.
16. Kimmoun A, Takagi K, Gall E, Ishihara S, Hammoum P, El Bèze N, Bourgeois A, Chassard G, Pegorer-Sfes H, Gayat E, Solal AC, Hollinger A, Merkling T, Mebazaa A; METAHF Team. Temporal trends in mortality and readmission after acute heart failure: a systematic review and meta-regression in the past four decades. Eur J Heart Fail. 2021 Mar;23(3):420-431.
17. Authors/Task Force Members:, McDonagh TA, Metra M, Adamo M, Gardner RS, Baumbach A, Böhm M, Burri H, Butler J, Čelutkienė J, Chioncel O, Cleland JGF, Coats AJS, Crespo-Leiro MG, Farmakis D, Gilard M, Heymans S, Hoes AW, Jaarsma T, Jankowska EA, Lainscak M, Lam CSP, Lyon AR, McMurray JJV, Mebazaa A, Mindham R, Muneretto C, Francesco Piepoli M, Price S, Rosano GMC, Ruschitzka F, Kathrine Skibelund A; ESC Scientific Document Group. 2021 ESC Guidelines for the diagnosis and treatment of acute and chronic heart failure: Developed by the Task Force for the diagnosis and treatment of acute and chronic heart failure of the European Society of Cardiology (ESC). With the special contribution of the Heart Failure Association (HFA) of the ESC. Eur J Heart Fail. 2022 Jan;24(1):4-131.
18. Kimmoun A, Cotter G, Davison B, Takagi K, Addad F, Celutkiene J, Chioncel O, Solal AC, Diaz R, Damasceno A, Duengen HD, Filippatos G, Goncalvesova E, Merai I, Metra M, Ponikowski P, Privalov D, Sliwa K, Sani MU, Voors AA, Shogenov Z, Mebazaa A. Safety, Tolerability and efficacy of Rapid Optimization, helped by NT-proBNP and GDF-15, of Heart Failure therapies (STRONG-HF): rationale and design for a multicentre, randomized, parallel-group study. Eur J Heart Fail. 2019 Nov;21(11): 1459-1467.
19. Cotter G, Davison B, Metra M, Sliwa K, Voors AA, Addad F, Celutkiene J, Chioncel O, Cohen Solal A, Diaz R, Damasceno A, Duengen HD, Filippatos G, Goncalvesova E, Merai I, Ponikowski P, Privalov D, Sani MU, Takagi K, Shogenov Z, Saidu H, Mebazaa A. Amended STRONG-HF study design. Eur J Heart Fail. 2021 Nov;23(11): 1981-1982.

## Claims

1. A method for assessing heart failure therapy in a patient who has been hospitalized for acute heart failure, said method comprising the steps of
a) determining the amount of a BNP-type peptide, such as NT-proBNP, in a baseline sample, wherein said baseline sample has been obtained prior to discharge of the patient from hospital,
b) determining the amount of the BNP-type peptide in at least one further sample wherein said at least one further sample has been obtained after discharge of the patient,
c) comparing the amount in the baseline sample determined in step a) to the amount in the at least one further sample determined in step b),
wherein an increase, an unchanged amount, or a decrease of less than 10% of the amount of the BNP-type peptide in the at least one further sample as compared to the amount in the baseline sample is indicative for a patient who is eligible to an increase of the dose of at least one diuretic, but not to an increase of the dose of one or more components of guideline directed medical therapy (GDMT).

2. A computer-implemented method for assessing heart failure therapy in a patient who has been hospitalized for acute heart failure, said method comprising the steps of
a) receiving, at a processing unit, a value for the amount of a BNP-type peptide in a baseline sample, wherein said baseline sample has been obtained prior to discharge of the patient from hospital,
b) receiving, at the processing unit, a value determining the amount of the BNP-type peptide in at least one further sample wherein said at least one further sample has been obtained after discharge of the patient,
c) carrying out at the processing unit a comparison of the amount in the baseline sample determined in step a) to the amount in the at least one further sample determined in step b),
wherein an increase, an unchanged amount, or a decrease of less than 10% of the amount of the BNP-type peptide in the at least one further sample as compared to the amount in the baseline sample is indicative for a patient who is eligible to an increase of the dose of at least one diuretic, but not to an increase of the dose of one or more components of guideline directed medical therapy (GDMT).

3. The method of claim 1 or 2, wherein the sample is a blood, serum, or plasma sample or interstitial fluid.

4. The method of any one of claims 1 to 3, wherein the baseline sample has been obtained from the patient within two days prior to the discharge of the patient from the hospital.

5. The method of any one of claims 1 to 4, wherein the at least one further sample has been obtained from the patient about 1, about 2, about 3, and/or about 6 weeks after discharge from hospital.

6. The method of any one of claims 1 to 5, wherein the BNP-type peptide is NT-proBNP or BNP, in particular NT-proBNP.

7. The method of any one of claims 1 to 6, wherein an increase of the amount of the BNP-type peptide in the at least one further sample of at least 10%, such as at least 20%, at least 30%, at least 40% or at least 50% as compared to the amount in the baseline sample is indicative for a patient who is eligible to an increase of the dose of at least one diuretic, but not to an increase of the dose of one or more components of the guideline directed medical therapy (GDMT).

8. The method of any one of claims 1 to 7, wherein the patient has preserved ejection fraction or wherein the patient has a reduced ejection fraction.

9. The method of any one of claims 1 to 8, wherein the guideline directed medical therapy is the administration of one or more of the following medicaments
i. at least one beta-blocker (BB);
ii. at least one angiotensin converting enzyme inhibitor (ACEi), at least one angiotensin receptor blocker (ARB), and/or at least one angiotensin receptor neprilysin inhibitor (ARNi), and/or
iii. at least one mineralocorticoid receptor antagonist (MRA), and/or
iv. at least one SGLT2 inhibitor.

10. The method of any one of claims 1 to 9, wherein a patient who is not eligible to an increase of the dose of guideline directed medical therapy (GDMT) is a patient in which the dose of guideline directed medical therapy is maintained or reduced.

11. *In vitro* use of
i) at least one BNP-type peptide or
ii) at least one detection agent for the at least one BNP-type peptide
in a baseline sample and in at least one further sample obtained from a patient who has been hospitalized for acute heart failure for assessing heart failure therapy in said patient,
wherein said baseline sample has been obtained prior to discharge of the patient from hospital, and said at least one further sample has been obtained after discharge of the patient.

12. The *in vitro* use of claim 11, wherein the detection agent for the at least one BNP-type peptide is at least one antibody which specifically binds to said at least one BNP-type peptide, such as at least one antibody which specifically binds to NT-proBNP.

13. The *in vitro* use of claim 12, wherein the antibody is monoclonal or polyclonal antibody.

14. A device for assessing heart failure therapy, said device comprising a processing unit, and a computer program including computer-executable instructions, wherein said instructions, when executed by the processing unit, cause the processing unit to perform the computer-implemented method according to any one of claims 2 to 10.

15. A method of treating a patient who has been hospitalized for acute heart failure, said method comprising the steps of
a) determining the amount of a BNP-type peptide, such as NT-proBNP, in a baseline sample, wherein said baseline sample has been obtained prior to discharge of the patient from hospital,
b) determining the amount of the BNP-type peptide in at least one further sample wherein said at least one further sample has been obtained after discharge of the patient,
c) comparing the amount in the baseline sample determined in step a) to the amount in the at least one further sample determined in step b),
d) identifying a patient as being eligible to an increase of the dose of at least one diuretic, but not to an increase of the dosage of guideline directed medical therapy (GDMT), preferably based the results of step c)
e) treating the identified patient with an increased dose of at least one diuretic, while maintaining the dose of one or more components of guideline directed medical therapy (GDMT).
